# EUROPEAN PATENT SPECIFICATION

(11) **EP 0 377 518 B1**
(45) Date of publication and mention of the grant of the patent: **07.02.1996**
(21) Application number: 90300130.3
(22) Date of filing: 05.01.1990
(51) Int. Cl.: A61K 31/485, A61K 9/52, A61K 9/54

(54) **Sustained release pharmaceutical composition**
Arzneimittel mit verzögerter Freigabe
Composition pharmaceutique à libération retardée

(30) Priority: 06.01.1989 AU 2192/89
(43) Date of publication of application: 11.07.1990
(60) Divisional application: 94200469.8
(73) Proprietor: F.H. FAULDING & CO. LIMITED, Parkside South Australia 5063 (AU)
(72) Inventor: Morella, Angel Mario, Campbelltown SA 5014 (AU); Fisher, Mark Christopher, Birkenhead SA 5014 (AU)
(74) Representative: Moon, Donald Keith

(56) References cited:
- EP-A- 0 164 959
- EP-A- 0 205 282
- EP-A- 0 260 236
- EP-A- 0 327 295

## Description

The present invention relates to a sustained release pharmaceutical composition, in particular a sustained release pharmaceutical composition including an active ingredient of high solubility in water, and to a method of preparing same.

As is known in the prior art, it is desirable in the treatment of a number of diseases, both therapeutically and prophylactically to provide the active pharmaceutical ingredient is a sustained release form. Desirably the sustained release provides a generally constant rate of release over an extended period. Whilst there is known in the prior art numerous sustained release formulations, the extension of sustained release regimens to active pharmaceutical ingredients of high solubility in water has been extremely limited. It has been found in the prior art that the high solubility in water of the active ingredient tends to generate a product which is susceptible to the phenomenon known as "dose dumping". That is, release of the active ingredient is delayed for a time but once release begins the rate of release is very high. Moreover, fluctuations tend to occur in the plasma concentrations of active ingredient which increase the likelihood of toxicity. Further, some degree of diurnal variation in plasma concentration of active ingredient has also been noted.

Prior art preparations may also suffer from other disadvantages, for example bioavailability of prior art preparations may be compromised by food. This is important since complex dosage regimens may lead to non-compliance.

EP 205282 discloses a sustained release oral pharmaceutical composition which comprises granules of a mixture of the active ingredient with a higher aliphatic alcohol and a hydrated water soluble hydroxy alkyl cellulose, the granules are coated with a mucosa adhesive cellulose such as hydroxypropyl cellulose.

EP 327295 discloses a pharmaceutical composition including coated granules of a tetracycline antibiotic, the coating comprises an enteric polymer and an acid soluble polymer which provides a low rate of release in the stomach but a substantially immediate rate of release in the small intestine.

EP 164959 describes a sustained release pharmaceutical composition for compounds which are substantially soluble at low pH but less so at intermediate pH. The formulation provides uniform release of the active ingredients as it passes through the stomach and gastric intestinal tract.

EP 260236 discloses a multi-unit-dose composition comprising L-dopa. The coating which may comprise three polymers is applied as separate layers and provides a very slow rate of release in the stomach at acid pH but rapid release at neutral pH.

Typical highly water soluble active ingredients include the opioid drugs which still play a major role in the treatment of acute and chronic pain, particularly pain associated with terminal diseases such as cancer.

Morphine is regarded as the opioid drug of choice in the treatment of cancer pain. It is universally acknowledged that the oral route of administration is preferred if sufficient pain relief can be obtained with an acceptable profile of side effects with respect to incidence and severity. Until recently, the liquid or immediate release tablet formulations of morphine were the only dosage forms available to physicians for oral administration in the treatment of cancer pain.

The oral administration of morphine has had many critics in the prior art who point to a supposed lack of efficacy. However, the accumulated evidence, particularly from the hospice environment, indicates that this criticism is unfounded if the dose and dosing interval are specifically optimised for each patient, the morphine doses are administered before the pain returns and in a strictly regular regimen. In practical terms, this means morphine doses ranging from 10 mg to in excess of 500 mg with dosing intervals ranging from every 2 to 6 hours. A relationship between blood morphine concentration and pain relief has been established in the treatment of post-operative and cancer pain.

The studies propose that there is a minimum effective concentration (MEC) for morphine for each patient. There is a five-fold interpatient variation in MEC in the treatment of post-operative pain and an even greater variation for cancer pain. This concept of a MEC for opioids has also been demonstrated for pethidine, methadone, fentanyl and ketobemidone. Repeated oral or parenteral doses produce fluctuating blood opioid concentrations, with the peak concentrations sometimes resulting in side effects, while the trough concentrations are usually associated with inadequate pain relief. Therefore, a formulation of morphine which reduces the fluctuations in blood opioid concentrations and has a longer duration of pain relief (e.g. a sustained release preparation) has widespread potential to improve pain relief in terminal care.

Currently, there is only one such preparation (MST Continus or MS Contin) being marketed world-wide. However, the combined pharmacokinetic and pharmacodynamic data suggest that this product is actually a delayed release formulation with some sustained release characteristics. While the manufacturers recommend a 12 hour dosing interval, extensive clinical experience suggests that an 8 hour interval is more realistic for continuous pain control.

Accordingly, it is an object of the present invention to overcome, or at least alleviate, one or more of the difficulties related to the prior art.

Accordingly, in a first aspect of the present invention there is provided a sustained release pharmaceutical pellet composition for administration to a patient at a predetermined dosage and interval which comprises a core element containing a therapeutically effective amount of at least one active ingredient having an aqueous solubility of at least 1 in 30 and a coating on said core element which comprises the following components:
(a) 1 to 85% by weight of a matrix polymer which is insoluble independent of pH.
(b) from 1 to 60% of an enteric polymer which is substantially insoluble at a pH of from 1 to 4, but which is soluble at a pH of from 6 to 7.5;
(c) from 1 to 60% of an acid-soluble compound soluble at a pH of from 1 to 4, sufficient to provide a slow rate of release of the active ingredient in the stomach;
said percentages being by weight based on the total weight of components (a), (b) and (c); the ratio of the components (a), (b) and (c) in said coating being effective such that a slow release of the active ingredient will occur in the stomach and a relatively constant faster release in the intestines such that blood levels of active ingredient are maintained within the therapeutic range over an extended period of time; the rate of release in the intestine being 1.2 to 3 times greater than in the stomach.

By "sustained release" as used herein we mean release of active ingredient at such a rate that blood levels are maintained within the therapeutic range but below toxic levels over an extended period of time e.g. 10 to 24 hours or greater. By "active ingredient of high water solubility" as used herein we mean pharmaceutically active, orally acceptable ingredients having an aqueous solubility of approximately 1 in 30 or above.

By "bioavailability" as used herein we mean the extent to which the active drug ingredient is absorbed from the drug product and becomes available at the site of drug action.

The active ingredients may be selected from antihistamines, antibiotics, antituberculosis agents, colinergic agents, antimuscarinics, sympathomimetics, sympatholytic agents, autonomic drugs, iron preparations, haemostatics, cardiac drugs, antihypertensive agents, vasodilators, non-steroidal antiinflammatory agents, opiate agonists, anticonvulsants, tranquilisers, stimulants, barbiturates, sedatives, expectorants, antiemetics, gastrointestinal drugs, heavy metal antagonists, antithyroid agents, genitourinary smooth muscle relaxants and vitamins. The invention is applicable to active ingredients of high solubility whether the solubility characteristics are pH dependent or pH independent.

Examples of active ingredients are set out in the table below.

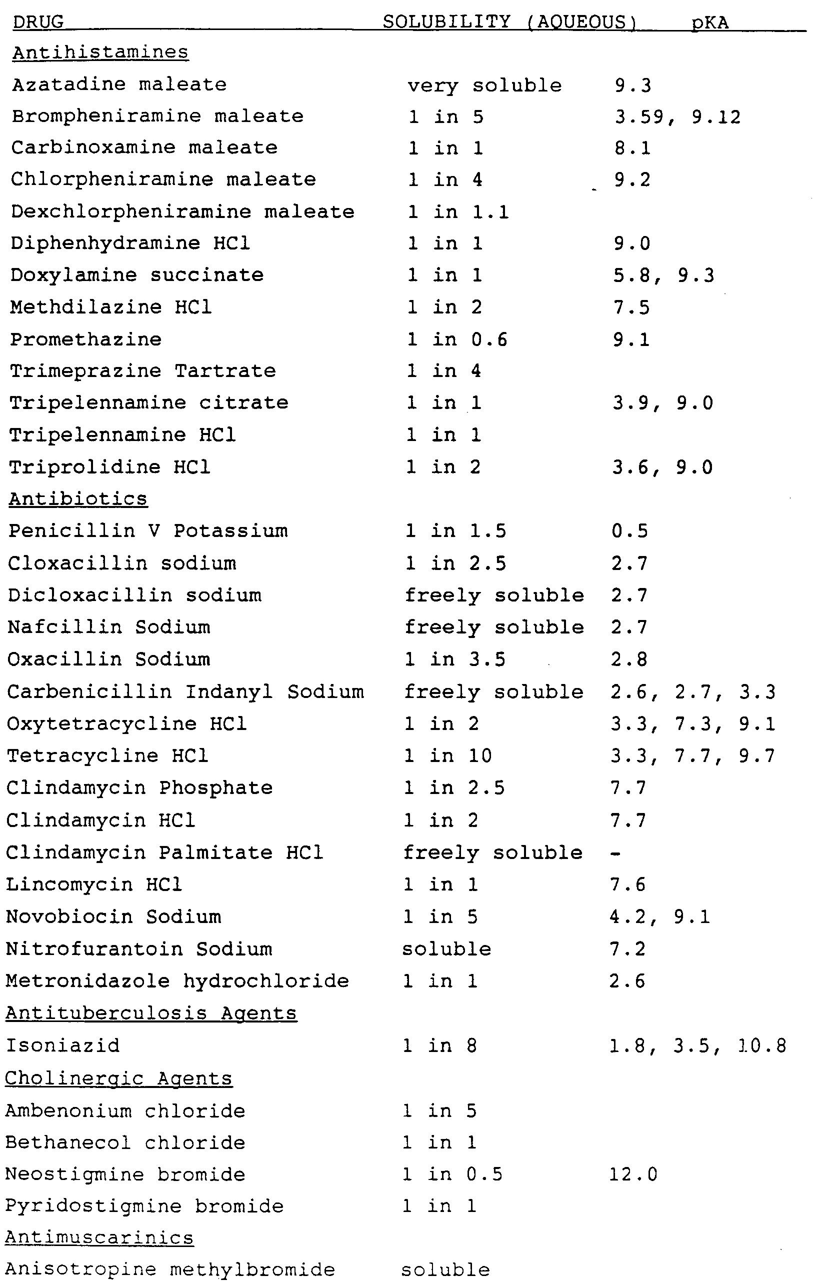

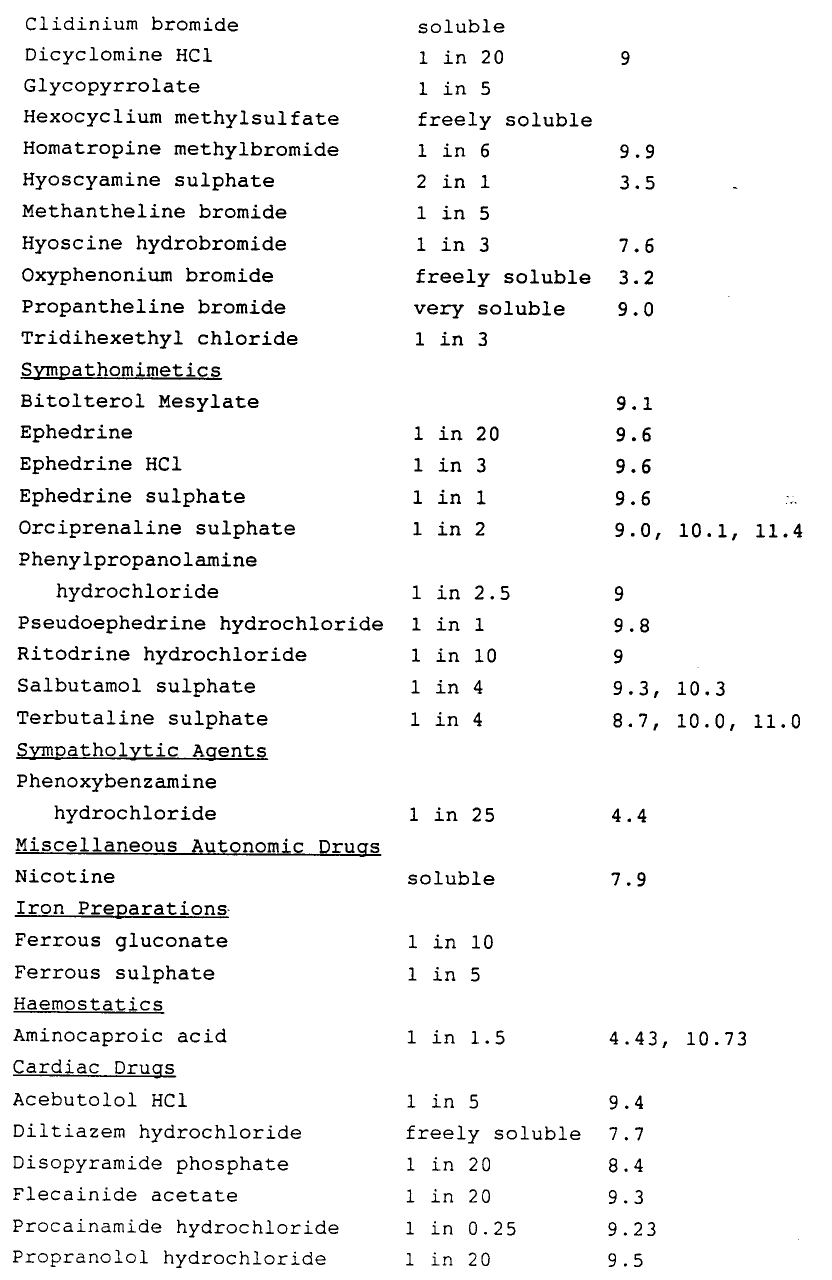

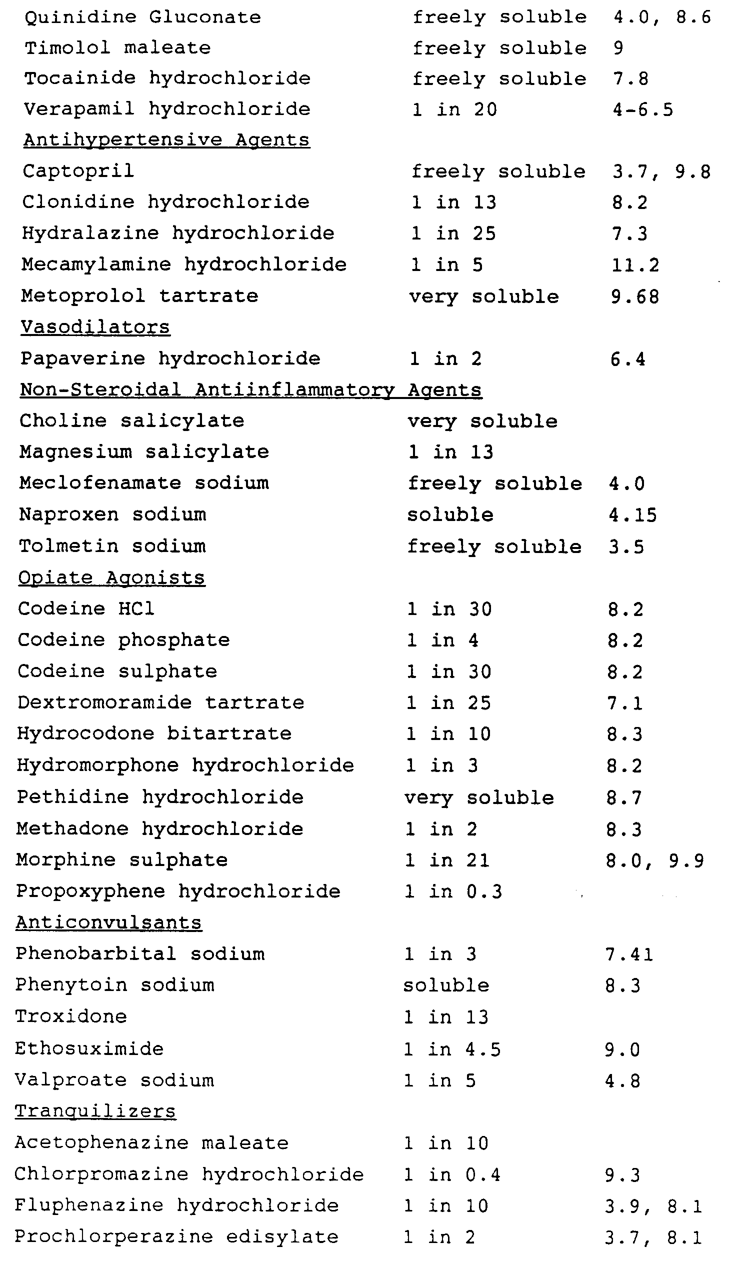

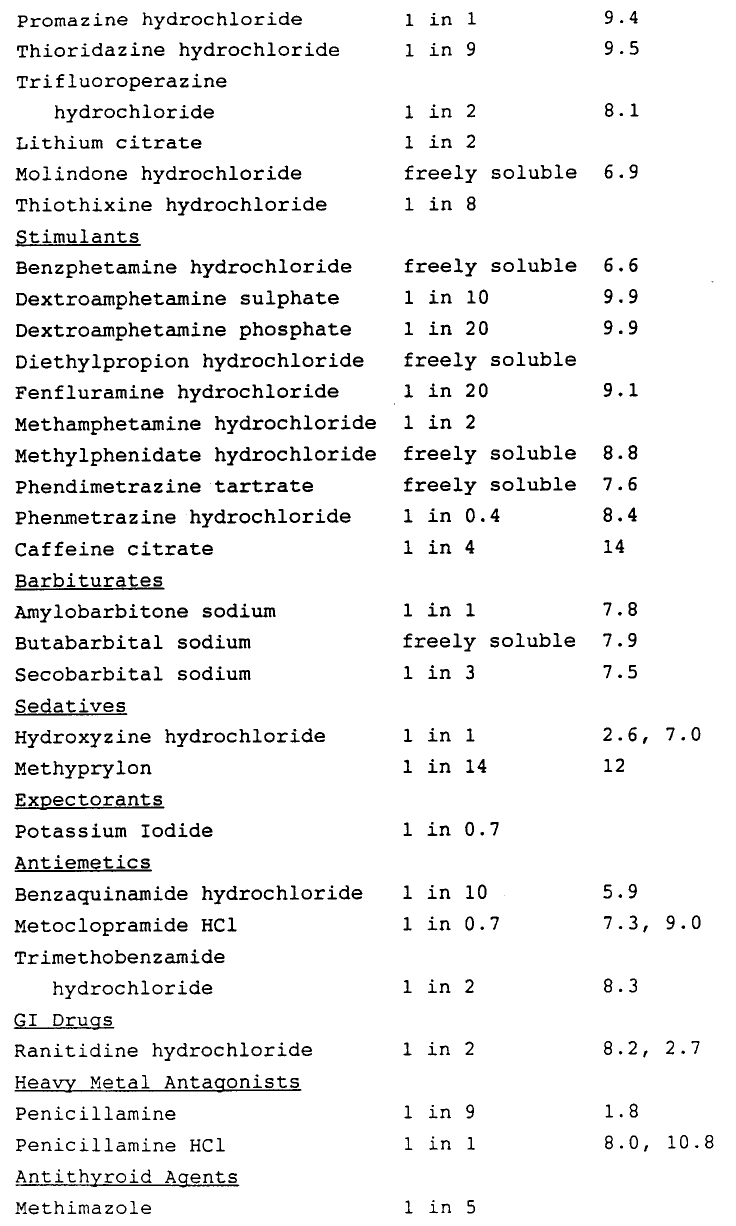

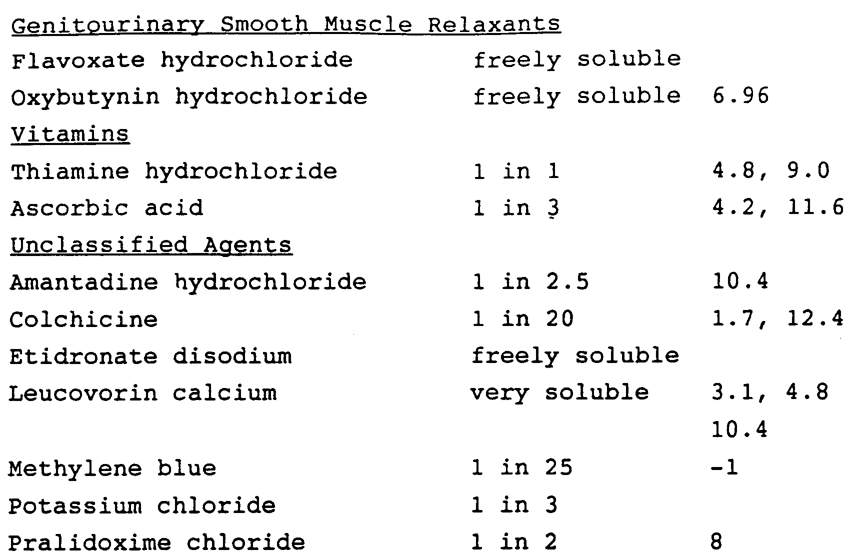

In the following description the active ingredient of high water solubility will be illustrated by reference to the opioid drug, morphine. However, this is illustrative only and the invention is in no way restricted thereto. Preferably, the active ingredient is an opiate selected from the group consisting of the salts of codeine, dextromoramide, hydrocodone, hydromorphine, pethidine, methadone, morphine and propoxyphene.

Morphine acts as an agonist primarily at mu, kappa and perhaps delta receptors in the central nervous system. By acting on these receptors the following pharmacological effects are seen. Analgesia due to a central action on pain perception, together with a modulatory effect on the central transmission of noxious sensation. It also causes drowsiness and euphoria (though sometimes dysphoria, particularly in those who are free of pain).

The pharmaceutical pellet composition according to the present invention may include a plurality of coated core elements.

The pharmaceutical composition may be provided in any suitable unit dosage form. An encapsulated form may be used.

The pharmaceutical pellet composition may be provided in a pellet or tableted pellet form. A tablet may be formed by compression of the pellets optionally with the addition of suitable excipients.

Preferably the core coating, in use, generates a dissolution profile for the sustained release composition, which is equal to or greater than the minimum dissolution profile required to provide substantially equivalent bioavailability to a capsule, tablet or liquid containing an equal amount of the at last one active ingredient in an immediately available form.

"Dissolution profile" as used herein, means a plot of amount of active ingredient released as a function of time. The dissolution profile may be measured utilising the Drug Release Test (724) which incorporates standard test USPXXII 1990. (Test(711)). A profile is characterised by the test conditions selected. Thus the dissolution profile may be generated at a preselected shaft speed, temperature and pH of the dissolution media.

A first dissolution profile may be measured at a pH level approximating that of the stomach. At least a second dissolution profile may be measured at pH levels approximating that of at least one point in the intestine.

A highly acidic pH may simulate the stomach and a less acidic to basic pH may simulate the intestine. By the term "highly acidic pH" as used herein we mean a pH in the range of approximately 1 to 4. By the term "less acidic to basic pH" we mean a pH of greater than 4 up to approximately 7.5, preferably approximately 6 to 7.5.

A pH of approximately 1.2 may be used to simulate the pH of the stomach.

A pH of approximately 6.0 to 7.5 preferably 7.5 may be used to simulate the pH of the intestine.

Accordingly in a preferred embodiment the invention provides a sustained release pharmaceutical pellet composition such that, the active ingredient has a first dissolution profile measured at a pH of from 1 to 4, and a second dissolution profile measured at a pH of about 7.5 and such that said first and second dissolution profile are each at least equal to the minimum dissolution required to provide substantially the same bio-availability as with an immediate release oral dosage form.

More preferably, the composition, in use, exhibits less fluctuations in plasma concentrations in active ingredient at steady state over a 24 hour period, relative to the active ingredient in an uncoated form and/or exhibits less diurnal variation in plasma concentration of active ingredient relative to known capsules or tablets containing the at least one active ingredient in a sustained release form.

For example, dissolution profiles have been generated which exhibit bioavailability substantially equivalent to, or better than, commercially known morphine compositions including MS Contin, MST Continus and morphine solution.

Accordingly in a preferred embodiment of the invention there is provided a sustained release pharmaceutical pellet composition for administration to a patient at a predetermined dosage and interval which comprises a core element containing a therapeutically effective amount of an acid addition salt of morphine and a coating on said core element which comprises the following components:
(a) 1 to 85% by weight of a matrix polymer which is insoluble independent of pH.
(b) from 1 to 60% of an enteric polymer which is substantially insoluble at a pH of from 1 to 4, but which is soluble at a pH of from 6 to 7.5;
(c) from 1 to 60% of an acid-soluble compound soluble at a pH of from 1 to 4, sufficient to provide a slow rate of release of the active ingredient in the stomach;
said percentages being by weight based on the total weight of components (a), (b) and (c); the ratio of the components (a), (b) and (c) in said coating being effective such that a slow release of the active ingredient will occur in the stomach and a relatively constant faster release in the intestines such that blood levels of active ingredient are maintained within the therapeutic range over an extended period of time; the rate of release in the intestine being 1.2 to 3 times greater than in the stomach.

It will be understood that further since the active ingredient is provided in a sustained release pellet form significantly less fluctuations in plasma concentrations of active ingredients at steady state over a 24 hour period are encountered, and may allow for less frequent dosing relative to the active ingredient in an uncoated form. This is expected to result in less toxic and more effective therapeutic activity.

Similarly, it has been found that the pharmaceutical pellet composition according to the present invention exhibits less diurnal variation in plasma concentrations of active ingredient than prior art preparations, for example when administered on a two, three or four times daily dosage regimen.

Moreover, the pharmaceutical pellet composition according to the present invention shows no evidence of dose dumping. The relative bioavailability of the active ingredient generated from the pharmaceutical pellet composition is not compromised by food so that compliance will improve as the product may be taken without regard to meals.

Moreover, since the core coating is partially soluble at an acidic pH, for example as encountered in the stomach of the patients, some slow release of active ingredient will occur in the stomach. The slow rate of release of active ingredient may also be at a relatively constant rate.

The active ingredient may be available for absorption even in regions of the gastrointestinal tract which are not sufficiently alkaline to dissolve the enteric core coating component.

Thus the active ingredient is available for absorption in an absorption region substantially immediately after the pyloric sphincter in the patient. Such an absorption region may generally be characterised by a pH between approximately 1.2 and 5.5. Absorption will occur in the small intestine but since absorption will continue over an extended period of time, thus some absorption will occur additionally some way into the large intestine.

Where the active ingredient of high solubility in water is a morphine compound, the morphine compound may take any suitable form. The morphine compound may be present in an anhydrous or hydrous form. The morphine compound may be provided in a salt form. Morphine sulphate is preferred. Morphine sulphate pentahydrate is particularly preferred.

Advantages of the sustained release pharmaceutical pellet composition according to the present invention may thus be summarised as follows
(i) The time during which morphine blood levels at steady state are greater than or equivalent to 75% of maximum blood levels (t>0.75Cₘₐₓ) may be approximately 3 hours or greater. Generally t>0.75Cₘₐₓ may be 3.5 hours or greater (t>0.75Cₘₐₓ for MS Contin has been reported to be is 3.5 hours).
(ii) peak to trough variations in blood morphine concentrations at steady state will be between 60-100% (these variations for MS Contin have been reported to be are approximately 300% and for Morphine Solution 4 hourly are approximately 200%)
(iii) diurnal variations may be reduced
(iv) the co-administration of food will not significantly decrease the extent of morphine absorption or alter the rate of morphine absorption when compared with administration in the fasted state the effect of food on morphine absorption from MS Contin is not known)
(v) inter- and intra-subject variation in blood morphine pharmacokinetics may be reduced.

Accordingly in a preferred embodiment of the present invention there is provided a sustained release pharmaceutical pellet composition such that, in use, the time during which the morphine blood levels at steady state are greater than or equivalent to 75% of maximum blood levels (t≧0.75Cmax) is approximately 3 hours or greater.

The core coating includes
at least one polymer which is substantially insoluble independent of pH (insoluble matrix polymer); and
1 to 85% by weight of a matrix polymer which is insoluble independent of pH.
from 1 to 60% of an enteric polymer which is substantially insoluble at a pH of from 1 to 4, but which is soluble at a pH of from 6 to 7.5;
from 1 to 60% of an acid-soluble compound soluble at a pH of from 1 to 4
It has been found necessary in order to achieve a slow rate of release at acidic pH for pH dependent or independent drugs, and faster relatively constant rate of release over an extended period of time to include the above three components in the hybrid core coating composition.

Preferably the enteric polymer is readily soluble at less acidic to basic pH (pH 6 to 7.5).

Preferably the at least partially soluble component is a readily water-soluble component.

The rate of dissolution at highly acidic pH of the core coating will depend on the amount of the at least one partially acid soluble component, the pH dependent and pH independent polymers, and the thickness of the coating. Typical core coatings may be in the range of approximately 5 to 200 um, preferably approximately 25 to 50 um. It will be understood, accordingly, that the rate of absorption may be modified by modifying the thickness and/or the composition of the hybrid core coating.

Once a minimum amount of the at least partially acid soluble component and/or the maximum thickness of the coating to achieve the minimum dissolution profile at an highly acidic pH has been established, then it is simply a matter of design choice to adjust the composition and/or thickness of coating as desired.

It has been found that the dissolution rate of the soluble drug at various pH's can be modified at will by altering the ratio of polymers. The ternary system of polymers according to the present invention allows greater flexibility than as known in prior art using only binary systems of polymers.

The at least one enteric polymer may be selected from cellulose acetate phthalate, hydroxypropyl methylcellulose phthalate (HPMCP), polyvinyl acetate phthalate, methacrylic acid copolymer, hydroxypropyl methylcellulose acetate succinate, shellac, cellulose acetate trimellitate and mixtures thereof. Particularly preferred enteric polymers include synthetic resin bearing carboxyl groups. The methacrylic acid: acrylic acid ethylester 1:1 copolymer sold under the trade designation "Eudragit L100-55" has been found to be suitable.

The at least one enteric polymer is present in the coating in an amount of from approximately 1 to 60% by weight, preferably 2 to 20% by weight, more preferably 5 to 15% by weight, based on the total weight of the hybrid core coating excluding weight of filler and plasticiser.

The at least partially acid-soluble component may be selected from polymers such as polyvinyl pyrrolidone, hydroxypropyl cellulose, hydroxypropyl methylcellulose, polyethylene glycol, polyvinyl alcohol and monomers therefor such as sugars, salts, or organic acids and mixtures thereof.

The at least partially acid-soluble component is present in the coating in amounts of from approximately 1 to 60%, preferably 15 to 40% by weight, more preferably 20 to 35% by weight, based on the total weight of the hybrid core coating excluding weight of filler and plasticiser.

The at least one insoluble matrix polymer may be any suitable pharmaceutically acceptable polymer substantially insoluble independent of pH. The polymer may be selected from ethylcellulose, acrylic and/or methacrylic ester polymers or mixtures thereof and the like may be used. Polymers or copolymers of acrylates or methacrylates having a low quaternary ammonium content may be used. The acrylic acid ethyl ester: methacrylic acid methyl ester (1:1) copolymer has been found to be suitable.

The at least one insoluble matrix polymer is present in the coating in an amount of from approximately 1 to 85% by weight preferably 35 to 75% by weight, more preferably 45 to 65% by weight, based on the total weight of the hybrid core coating excluding weight of filler and plasticiser.

The core coating may further include at least one plasticiser; and optionally
at least one filler.

Accordingly in a preferred aspect the core coating includes
0 to approximately 50% by weight, preferably 2.5 to 30% by weight, based on the total weight of the core coating of at least one plasticiser selected from diethyl phthalate, triethyl citrate, triethyl acetyl citrate, triacetin, tributyl citrate, polyethylene glycol and glycerol and the like; and
0 to approximately 75% by weight based on the total weight of the hybrid core coating of a filler selected from insoluble materials such as silicon dioxide, titanium dioxide, talc, alumina, starch, kaolin, polacrilin potassium, powdered cellulose, and microcrystalline cellulose and mixtures thereof.

The at least one plasticiser may be selected from diethyl phthalate, triethyl citrate, triethyl acetyl citrate, triacetin, tributyl citrate, polyethylene glycol and glycerol and the like. It will be understood that the plasticiser used may be largely dictated by the polymer used in the coating formulation, and the compatibility of the plasticiser with coating solution or dispersion. It should be noted that acid or water soluble plasticisers can also be used to function as the partially acid soluble component. The plasticiser may function to improve the physical stability of the core coating. A plasticiser is particularly preferred where the polymer has a high glass transition temperature and/or is of a relatively low molecular weight.

The plasticiser may be present in any suitable effective amount. Amounts of from approximately 0 to 50% by weight preferably 2.5 to 30% by weight based on the total weight of the hybrid core coating, have been found to be suitable.

The filler may be present in any suitable effective amount. Amounts of from 0 to approximately 75% by weight, preferably 15 to 60% by weight, more preferably 25 to 45% by weight, based on the total weight of the hybrid core coating have been found to be suitable.

Accordingly in a further preferred aspect the hybrid core coating has a formulation

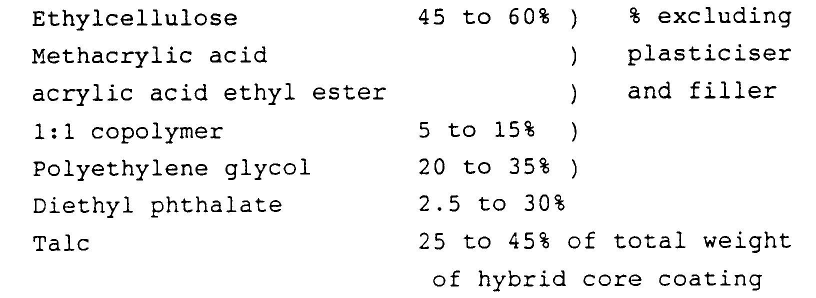

In a preferred aspect of the present invention the core element of the pharmaceutical composition according to the present invention may include an effective amount of
at least one active ingredient of high solubility;
at least one core seed; and
at least one binding agent.

The active ingredient may be present in any suitable effective amount. The amount of active ingredient is dependent on the potency of the active ingredient and on the desired dosage strength and volume of a unit dose of the drug product. The active ingredient may be present in amounts of approximately 0.1 to 94.9 by weight, based on the total weight of the core element. The active ingredient may preferably be a morphine compound. The morphine compound may be present in amounts of approximately 10 to 60% by weight, based on the total weight of the core element.

The binding agent may be present in amounts of from approximately 0.1 to 45% by weight preferably approximately 0.1 to 20% by weight based on the total weight of the core element.

The binding agent may be of any suitable type. Suitable binders may be selected from polyvinyl pyrrolidone, hydroxypropyl cellulose, hydroxypropyl methylcellulose, methylcellulose and hydroxyethyl cellulose, sugars and mixtures thereof. The binding agent may be provided in the form of a granulating solution. An aqueous or organic solvent may be included. Methanol, ethanol or mixtures thereof may be used as solvents.

The size and amount of the core seed may vary substantially from approximately 100 um to 1700 um depending upon the amount of active ingredient to be included. Accordingly, the core seeds may vary from approximately 5 to 99% by weight, preferably 40 to 90% by weight based on the total weight of the core element, depending on the potency of the active ingredient. The core seed may be of such a diameter to provide a final core element having a diameter of approximately 200 to 2000 um.

The core seed may be of any suitable type. A sugar or an active core seed may be used.

The core element may further include other carriers or excipients, fillers, stabilizing agents and colorants. Suitable fillers may be selected from insoluble materials such as silicon dioxide, talc, titanium dioxide, alumina, starch, kaolin, polacrilin potassium, powdered cellulose, and microcrystalline cellulose and mixtures thereof. Soluble fillers may be selected from mannitol, sucrose, lactose, dextrose, sodium chloride, sorbitol and mixtures thereof.

In a preferred embodiment the core element includes an effective amount of at least one morphine compound; optionally
at least one core seed; and
at least one binding agent.

The core element may have a formulation

| | |
|---|---|
| Morphine sulphate | 10 to 60% by weight |
| Core seeds | 30 to 89.9% by weight |
| Hydroxypropylmethylcellulose | 0.1 to 10% by weight |

Alternatively the core element may have a formulation

| | |
|---|---|
| Morphine sulphate | 10 to 60% by weight |
| Core seeds | 30 to 87.5% by weight |
| Polyvinyl pyrrolidone | 2.5 to 10% by weight |

The hybrid core coating composition may be provided in the form of a solution, dispersion or suspension.

In the form of a solution, the solvent may be present in amounts of from approximately 25 to 97% by weight, preferably 85 - 97%, based on the total weight of the hybrid core coating composition. The solvent for the polymer may be a solvent such as water, methanol, ethanol, methylene chloride and mixtures thereof.

In the formof a dispension or suspension, the diluting medium may be present in amounts of from approximately 25 to 97% by weight, preferably 75 - 97%, based on the total weight of the hybrid core coating composition and is comprised predominantly of water.

Typical hybrid core coating formulations may be prepared in the amounts as follows:

### Core Coating Formulation

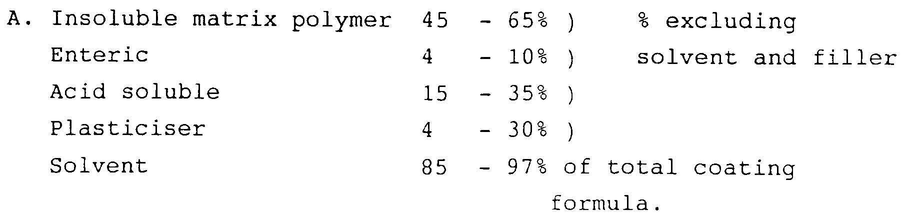

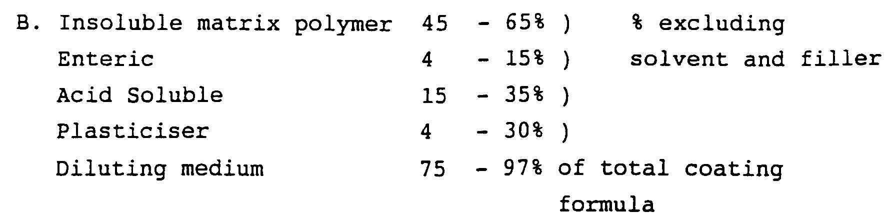

Optionally, an amount of filler not exceeding 50% of the core coating formulations weight excluding solvent, may be added.

In a second aspect the present invention provides a method for preparing a sustained release pharmaceutical pellet composition, which method comprises providing
a core element including at least one active ingredient having an aqueous solubility of at least 1 in 30; and
a core coating composition comprising a solution , suspension or dispersion of
(a) 1 to 85% by weight of a matrix polymer which is insoluble independent of pH.
(b) from 1 to 60% of an enteric polymer which is substantially insoluble at a pH of from 1 to 4, but which is soluble at a pH of from 6 to 7.5;
(c) from 1 to 60% of an acid-soluble compound soluble at a pH of from 1 to 4, sufficient to provide a slow rate of release of the active ingredient in the stomach;
said percentages being by weight based on the total weight of components (a), (b) and (c);
introducing the core element into a fluidised bed reactor; and
spraying the core coating composition onto the core element;
the ratio of the components (a), (b) and (c) in said coating being effective such that a slow release of the active ingredient will occur in the stomach and a relatively constant faster release in the intestines such that blood levels of active ingredient are maintained within the therapeutic range over an extended period of time; the rate of release in the intestine being 1.2 to 3 times greater than in the stomach.

In a preferred embodiment the method may further include the preliminary steps of providing
at least one active ingredient;
at least one binding agent;
at least one core seed; and
coating the core seed with the active ingredient and binding agent to form a core element.

In an alternative form the at least one binding agent is provided in a granulating solution. In this form the coating step may be conducted as a spheronisation process. The spheronisation process includes contacting the core seeds with the active ingredient and simultaneously adding the granulating solution thereto. The spheronisation process may be conducted in a spheronising machine.

In a further preferred embodiment the method may include the preliminary steps of providing
at least one active ingredient;
at least one binding agent; and
an effective amount of a solvent,
mixing the ingredients; and
subjecting the ingredients to an extrusion followed by marumerisation to form a core element.

The solvent may be an aqueous or organic solvent or mixtures thereof. The solvent may be present in an amount effective to allow the ingredients to be extruded.

The core elements formed are then subjected to a drying step. The drying step may be conducted in a fluidised bed or drying oven.

In a preferred form the at least one binding agent and active ingredient are provided in a solution or slurry. In this form the core seeds are sprayed with the solution or slurry. The spraying step may be conducted in any suitable coating equipment. The coating equipment may be a fluidised bed chamber, preferably a rotary fluid bed machine.

Spray coating of core elements may be undertaken utilising bottom, top or tangentially located spray nozzles. A bottom spray nozzle may reside proximate to the base of the fluidised bed facing upwards while a top spraying nozzle is located above the contents of the bed and facing downwards. The spray nozzle may reside in the mid-section of the fluidised bed and be orientated such as to spray tangentially to the rotating core elements.

In a preferred embodiment the active ingredient is a morphine compound, preferably a morphine salt.

The pharmaceutical pellet composition incorporating morphine compound may provide effective pain relief with twice or three times or four times daily administration. Versatility of dosing may be achieved with 10 mg, 20 mg, 50 mg, 100 mg, 200 mg, 500 mg or any other dose strength of capsules required.

The pharmaceutical pellet composition may be in multi-pellet encapsulated, sprinkle sachet or tableted forms.

Sustained release pharmaceutical pellet compositions according to the present invention are applicable for use in treating pain-associated conditions particularly the treatment of acute and chronic pain, particularly pain associated with terminal disease such as cancer and chronic backpain, as well as post-operative pain.

In a preferred embodiment the sustained release pharmaceutical pellet composition is provided in a unit dosage form which delivers an effective dose of active ingredient for a period of 8 to 24 hours.

The present invention will now be more fully described with reference to the accompanying examples. It should be understood, however, that the following description is illustrative only and should not be taken in any way as a restriction on the generality of the invention specified above.

### EXAMPLE 1

### 1. Formulation 1

### Core Composition 1

| | |
|---|---|
| Morphine Sulphate pentahydrate | 194 g |
| Core seeds | 170 g |
| Polyvinyl pyrrolidone | 37 g |
| Ethanol | 185 g |

### Hybrid Core Coating Composition 1

| | |
|---|---|
| Polyethylene Glycol | 12 g |
| Ethylcellulose | 25 g |
| Diethyl phthalate | 2 g |
| Methacrylic acid : acrylic acid ethyl ester 1:1 copolymer | 5 g |
| Talc | 22 g |
| Ethanol | 667 g |

### 2. Formulation 2

### Core Composition 2

| | |
|---|---|
| Morphine Sulphate pentahydrate | 194 g |
| Core Seeds | 170 g |
| Polyvinyl pyrrolidone | 37 g |
| Ethanol | 185 g |

### Hybrid Core Coating Composition 2

| | |
|---|---|
| Polyethylene Glycol | 25 g |
| Ethylcellulose | 41 g |
| Diethyl phthalate | 3 g |
| Methacrylic acid: acrylic acid ethyl ester 1:1 copolymer | 4 g |
| Talc | 37 g |
| Ethanol | 1106 g |

### 3. Formulation 3

### Core Composition 3

| | |
|---|---|
| Morphine Sulphate Pentahydrate | 364 g |
| Core Seeds | 733 g |
| Hydroxypropylmethylcellulose | 14 g |
| Ethanol | 986 g |

### Hybrid Core Coating Composition 3

| | |
|---|---|
| Polyethylene Glycol | 47 g |
| Ethylcellulose | 90 g |
| Diethyl phthalate | 19 g |
| Methacrylic acid : acrylic acid ethyl ester 1:1 copolymer | 20 g |
| Talc | 88 g |
| Ethanol | 2509 g |

### Spheronised Core Manufacture (Core Composition 1 and 2)

The core seeds were placed in a spheroniser. The core seeds were then coated with a dry mixture of the active ingredients and inactive excipients whilst concomittantly adding a solution of the binder components.

The wet cores so formed were then dried in a fluidised bed dryer for 1 hour.

### Rotacoating Core Manufacture (Core Composition 3)

The core seeds were placed in a rotor fluid bed machine. The core seeds were then coated with a suspension or solution of the active ingredients and inactive excipients including at least one binding agent, in a suitable liquid. The wet cores so formed were then dried in a suitable drier for one hour.

### Pellet Manufacture

(a) The dried spheronised cores 1 and 2 were then placed in a fluid bed coating apparatus. The hybrid core coating compositions 1 and 2 were then sprayed onto the cores 1 and 2 to form Formulation 1 and 2 pellets respectively. At the conclusion of the process, the pellets were fluid bed dried.
(b) The dried cores 3 were then placed in a rotary fluid bed or conventional fluid bed coating apparatus. The hybrid core coating composition 3 was then sprayed onto the cores 3 to form Formulation 3 pellets.

A dissolution test was conducted on the pellet compositions 1, 2 and 3 utilising the test method USPXXII 1990 (Test 711). A sample is dissolved in an aqueous medium previously degassed and equilibrated to 37°C. The media are USP pH 1.2 media without enzymes and pH 7.5 phosphate buffer. A sample of known volume is withdrawn at designated time intervals from the bath as directed and subjected to a suitable assay procedure. The mg of morphine sulphate as a function of time is plotted as the dissolution profile.

The tests were conducted at pH 1.2 and pH 7.5.

The baskets containing the samples were rotated at approximately 50 r.p.m. and the aqueous medium maintained at approximately 37°C.

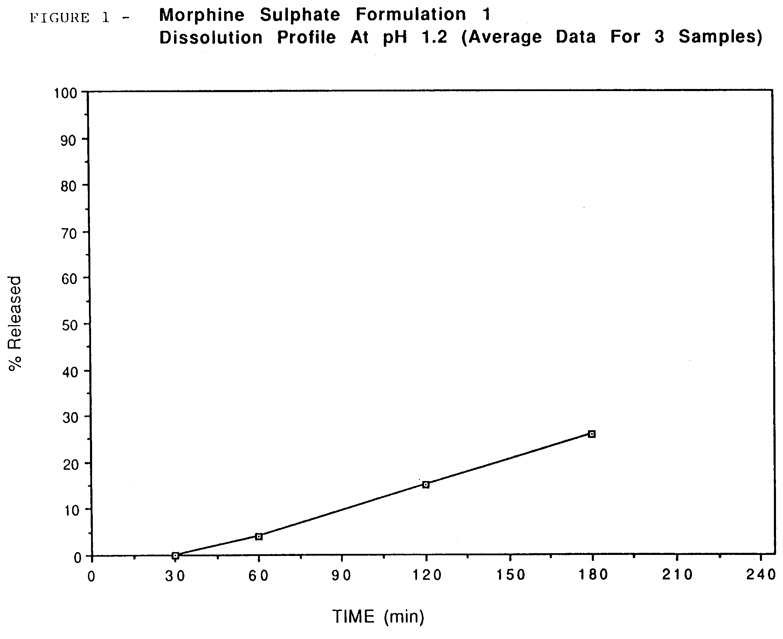

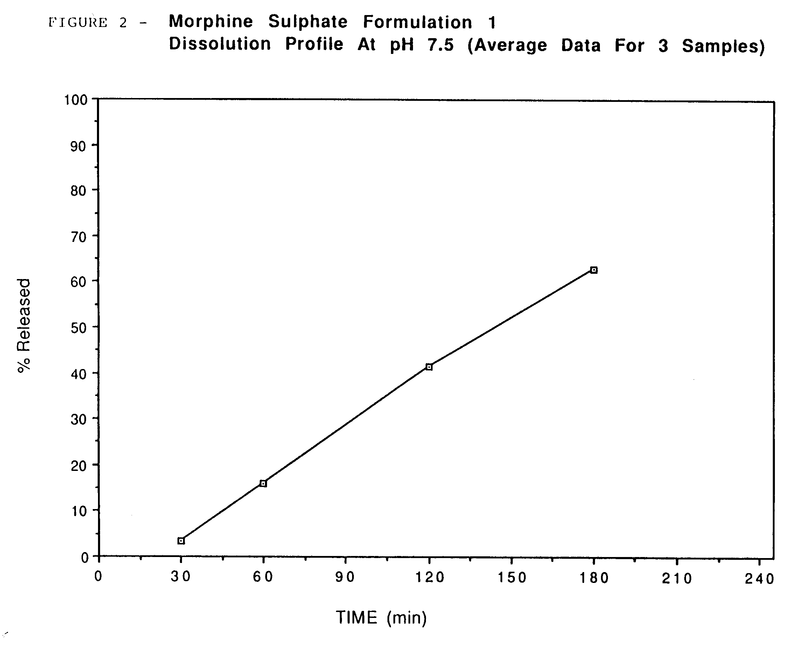

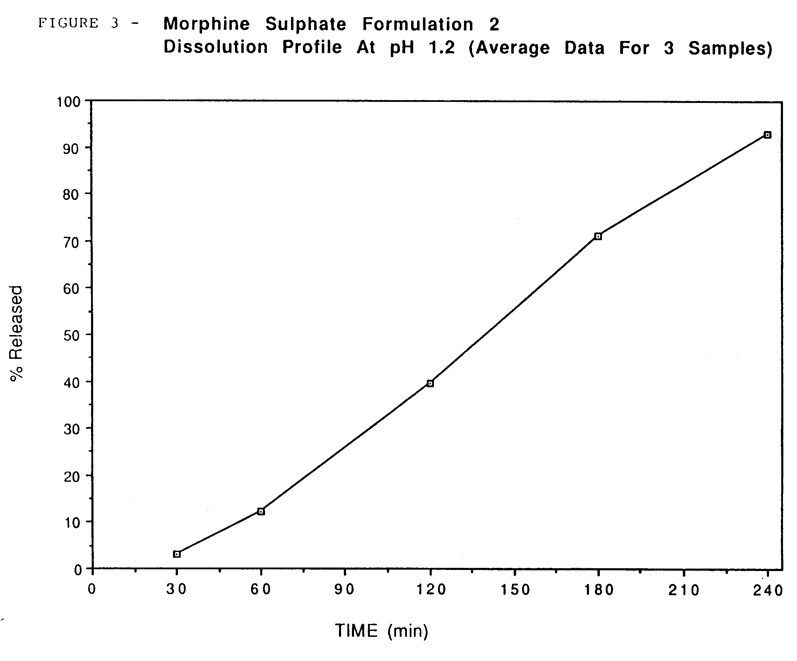

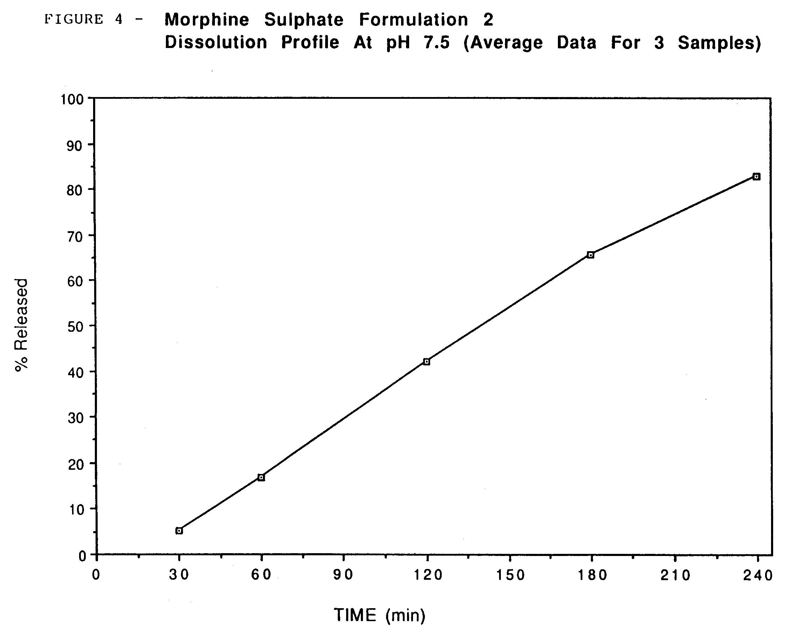

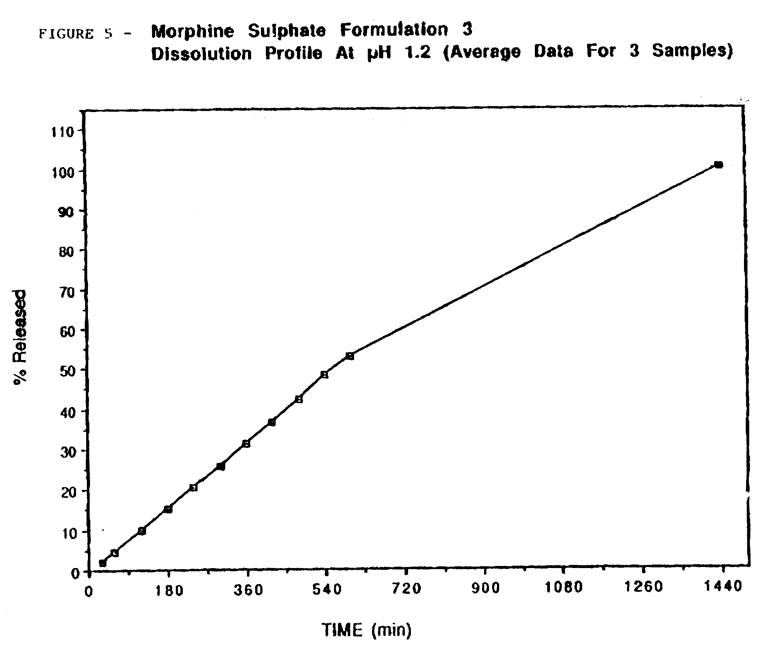

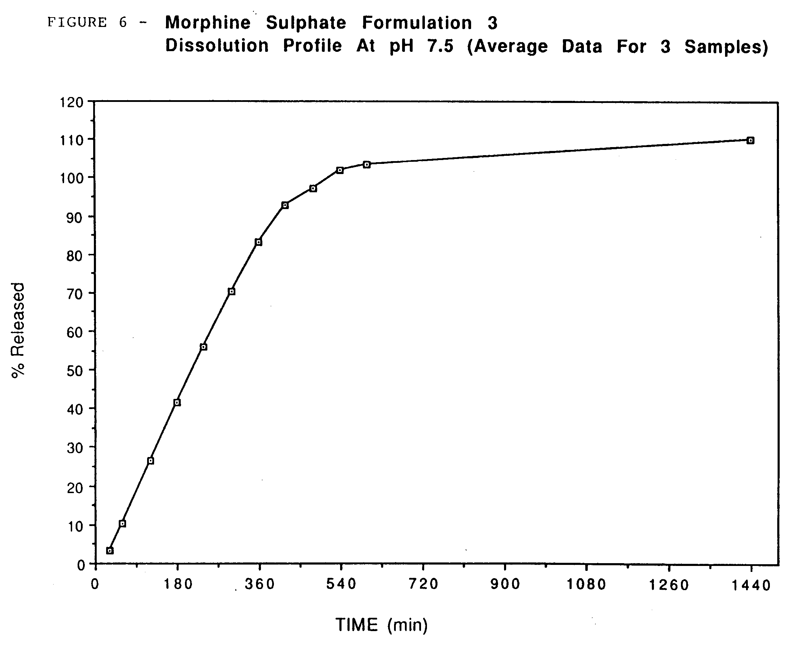

The results are given in Tables 1 to 6 and Figures 1 and 6 herein. The results for Formulation 1 at pH 1.2 and 7.5 are given in Tables 1 and 2 respectively. The hybrid Coating on Formulation 1 pellet allows dissolution at pH 1.2, a significantly faster rate of dissolution is observed at pH 7.5. The results for Formulation 2 pellet at pH 1.2 and 7.5 are given in Tables 3 and 4 respectively, and are similar to those obtained from Formulation 1.

The results for Formulation 3 pellets are similar to those achieved for Formulation 1 at pH 7.5. The results achieved for Formulation 3, however, illustrate a significant prolongation of release thereover.

**TABLE 1**

| DISSOLUTION DATA FOR FORMULATION 1 AT pH 1.2 (AVERAGED DATA FOR 3 SAMPLES) | | | | |
|---|---|---|---|---|
| TIME MIN. | MG RELEASED | SD | % RELEASED | SD |
| 30 | 0.00 | 0.00 | 0.00 | 0.00 |
| 60 | 2.29 | 0.09 | 4.04 | 0.15 |
| 120 | 8.43 | 0.18 | 14.88 | 0.28 |
| 180 | 14.66 | 0.39 | 25.87 | 0.71 |

**TABLE 2**

| DISSOLUTION DATA FOR FORMULATION 1 AT pH 7.5 (AVERAGED DATA FOR 3 SAMPLES) | | | | |
|---|---|---|---|---|
| TIME MIN. | MG RELEASED | SD | % RELEASED | SD |
| 30 | 1.85 | 0.09 | 3.28 | 0.17 |
| 60 | 9.03 | 0.25 | 16.07 | 0.45 |
| 120 | 23.20 | 0.42 | 41.29 | 0.77 |
| 180 | 35.39 | 0.50 | 63.00 | 1.01 |

**TABLE 3**

| DISSOLUTION DATA FOR FORMULATION 2 AT pH 1.2 (AVERAGED DATA FOR 3 SAMPLES) | | | | |
|---|---|---|---|---|
| TIME MIN. | MG RELEASED | SD | % RELEASED | SD |
| 30 | 1.64 | 0.00 | 3.22 | 0.01 |
| 60 | 6.26 | 0.09 | 12.25 | 0.16 |
| 120 | 20.24 | 0.18 | 39.63 | 0.46 |
| 180 | 36.39 | 0.27 | 71.27 | 0.72 |
| 240 | 47.47 | 0.49 | 92.97 | 1.12 |

**TABLE 4**

| DISSOLUTION DATA FOR FORMULATION 2 AT pH 7.5 (AVERAGED DATA FOR 3 SAMPLES) | | | | |
|---|---|---|---|---|
| TIME MIN. | MG RELEASED | SD | % RELEASED | SD |
| 30 | 2.63 | 0.00 | 5.12 | 0.03 |
| 60 | 8.69 | 0.09 | 16.94 | 0.11 |
| 120 | 21.62 | 0.33 | 42.13 | 0.40 |
| 180 | 33.66 | 0.59 | 65.60 | 0.79 |
| 240 | 42.47 | 0.82 | 82.78 | 1.13 |

**TABLE 5**

| DISSOLUTION DATA FOR FORMULATION 3 AT pH 1.2 (AVERAGED DATA FOR 3 SAMPLES) | | | | |
|---|---|---|---|---|
| TIME MIN. | MG RELEASED | SD | % RELEASED | SD |
| 30 | 1.44 | 0.39 | 2.12 | 0.53 |
| 60 | 3.03 | 0.33 | 4.48 | 0.39 |
| 120 | 6.78 | 0.30 | 10.03 | 0.36 |
| 180 | 10.17 | 0.18 | 15.04 | 0.34 |
| 240 | 13.87 | 0.41 | 20.51 | 0.29 |
| 300 | 17.45 | 0.31 | 25.81 | 0.30 |
| 360 | 21.29 | 0.21 | 31.49 | 0.27 |
| 420 | 24.75 | 0.32 | 36.62 | 0.46 |
| 480 | 28.60 | 0.64 | 42.30 | 0.37 |
| 540 | 32.63 | 0.42 | 48.28 | 0.45 |
| 600 | 35.80 | 0.92 | 52.95 | 0.37 |
| 24 hours | 67.60 | 1.26 | 100.04 | 3.79 |

**TABLE 6**

| DISSOLUTION DATA FOR FORMULATION 3 AT pH 7.5 (AVERAGED DATA FOR 3 SAMPLES) | | | | |
|---|---|---|---|---|
| TIME MIN. | MG RELEASED | SD | % RELEASED | SD |
| 30 | 2.19 | 0.11 | 3.23 | 0.17 |
| 60 | 7.05 | 0.89 | 10.38 | 1.26 |
| 120 | 18.07 | 1.05 | 26.63 | 1.44 |
| 180 | 28.12 | 1.03 | 41.44 | 1.35 |
| 240 | 37.86 | 1.05 | 55.80 | 1.32 |
| 300 | 47.60 | 1.48 | 70.16 | 1.96 |
| 360 | 56.33 | 0.54 | 83.03 | 0.47 |
| 420 | 63.03 | 2.01 | 92.90 | 2.76 |
| 480 | 65.97 | 0.61 | 97.23 | 0.75 |
| 540 | 69.13 | 0.41 | 101.89 | 0.79 |
| 600 | 70.20 | 0.43 | 103.47 | 0.45 |
| 24 hours | 74.76 | 2.36 | 110.19 | 3.04 |
| SD = Standard Deviation | | | | |

### EXAMPLE 2

Two sustained release morphine compositions in accordance with the present invention have been trialled in patients with back pain (fed and fasting) and in healthy volunteers (fasting). The results of these trials suggest that Faulding already has a product that is superior to a commercial product MS Contin with regard to sustained delivery of morphine. An investigation has also been initiated into understanding the effect that food has on the absorption of morphine.

The sustained release oral morphine compositions according to the present invention are designated Formulation 1 and Formulation 2.

### 1. PART A

A single dose 3 way crossover study under fasted conditions was conducted in six patients suffering chronic pain. On 3 occasions separated by one week, patients received a 50 mg oral morphine dose as either a solution (reference dose) or one of two sustained release formulations as pellets contained within a capsule (designated Formulation 1, a pH dependent release formulation; and Formulation 2, a pH independent release formulation). The doses were administered after an overnight fast. Venous blood samples were taken at specified time intervals from immediately after dose administration for 30 hours after the sustained release formulations and for 10 hours after the reference solution dose. The morphine concentration in the blood samples was quantitated using high pressure liquid chromatography (HPLC) with electrochemical detection. Table 3.1 summarises the mean area under the curve (AUC); Cₘₐₓ (maximum blood concentration); Tₘₐₓ (time to reach peak blood concentration); T_{1/2} (apparent terminal half life); T_{≧0.75 Cmax} (time for which blood concentration was greater than 75% of Cₘₐₓ) and relative bioavailability (F%).

The results revealed that both Formulation 1 and Formulation 2 provide a sustained release relative to the reference solution as assessed by:
(1) a lower Cₘₐₓ for the formulations;
(2) a longer Tₘₐₓ for the formulations; and
(3) a longer time for which the blood morphine concentration was above 0.75 Cₘₐₓ for the formulations.

There was a significant decrease in Cₘₐₓ values for each formulation compared with the reference solution. The mean (±SD) Cₘₐₓ for the solution was 73.6±30.9 ng/mL whereas the corresponding values for Formulation 1 and Formulation 2 were 21.6±7.1 ng/mL and 23.2±4.8 ng/mL respectively. The variability in Cₘₐₓ for Formulations 1 and 2 as demonstrated by the coefficient of variation was significantly less than that of the solution in the same patients.

There was a significant increase in Tₘₐₓ values for the formulations relative to that obtained with the reference solution. The mean (±SD) Tₘₐₓ for solution was 1.07±1.09 hours whereas the equivalent values were 5.33±1.2 hours and 4.25±1.33 hours for Formulations 1 and 2 respectively. The variability in Tₘₐₓ values for the formulations was less than that obtained for the solution in the same patients.

The time the blood morphine concentration was greater than or equal to 0.75 Cₘₐₓ was significantly greater for the formulations compared to the reference solution dose. The mean time was 190 minutes for Formulation 1 and 237 minutes for Formulation 2 compared to only 59 minutes for the reference solution. Expressing these data as percentage of the time of the reference solution, Formulation 1 was 322% while Formulation 2 has 400% greater time that the blood morphine concentration was greater than 0.75 Cₘₐₓ compared to the solution.

There was no significant difference between the AUC for the formulations and that obtained for the reference solution (Table 3.1).

The relative bioavailability for the formulations was calculated from the ratio of the AUC for the appropriate formulation relative to that obtained for the reference solution for each patient. The relative bioavailability was 83.5% for Formulation 1 and 102.6% for Formulation 2.

The AUC and relative bioavailability data suggest that the extent of absorption of morphine from the three different formulations is similar whereas the Cₘₐₓ, Tₘₐₓ and T_{≧0.75Cmax} data indicate that the formulations exhibit the typical slower and prolonged absorption of a true sustained release preparation.

**TABLE 3.1**

| RESULT OF STUDY PART A | | | | | |
|---|---|---|---|---|---|
| PARAMETER | SOLUTION MEAN | FORMULATION 1 | | FORMULATION 2 | |
| | | MEAN | OBSERVED DIFF | MEAN | OBSERVED DIFF |
| AUC (ng.h/mL) | 199.77 | 170.72 | -29.05 | 193.77 | -6.0 |
| SD | ±66.32 | ±86.3 | | ±46.35 | |
| CV% | 33 | 51 | | 24 | |
| Cₘₐₓ (ng/mL) | 73.57 | 21.60 | -52.0 | 23.16 | -50.4 |
| SD | ±30.92 | ±7.12 | | ±4.76 | |
| CV% | 42 | 33 | | 21 | |
| Tₘₐₓ (hours) | 1.07 | 5.33 | 4.26 | 4.25 | 3.18 |
| SD | ±1.1 | ±1.21 | | ±1.33 | |
| CV% | 103 | 23 | | 31 | |
| Bioavailability (F%) | 100.0 | 83.53 | -16.47 | 102.62 | 2.62 |
| SD | ±0.00 | ±27.87 | | ±25.77 | |
| CV% | 0 | 33 | | 25 | |
| t_{1/2} (hours) | 3.02 | 6.58 | 3.56 | 7.65 | 4.63 |
| SD | ±1.97 | ±5.33 | | ±5.59 | |
| CV% | 65 | 81 | | 73 | |
| T_{≧0.75 Cmax} (minutes) | 59.0 | 189.8 | 130.8 | 237.3 | 178.3 |
| SD | ±37 | ±76 | | ±95 | |
| CV% | 63 | 40 | | 40 | |

### 2. PART B

A single dose 3 way crossover study under fed conditions was conducted in six patients suffering chronic pain. The same patients took part in both Parts A and B of this study. On 3 occasions separated by one week, patients received a 50 mg oral morphine dose as either a solution (reference dose) or one of two sustained release formulations as pellets contained within a capsule (designated Formulation 1, a pH dependent release formulation; and Formulation 2, a pH independent release formulation). The doses were administered after an overnight fast. Venous blood samples were taken at specified time intervals from immediately after dose administration for 30 hours after the sustained release formulations and for 10 hours after the reference solution dose. The morphine concentration in the blood samples was quantitated using high pressure liquid chromatography (HPLC) with electrochemical detection. Table 3.2 summarises the mean area under the curve (AUC); Cmax (maximum blood concentration); Tₘₐₓ (time to reach peak blood concentration); T_{≧0.75 Cmax} (time for which blood concentration was greater than 75% of Cₘₐₓ) and relative bioavailability (F%).

The results revealed that, in the presence of food, both Formulation 1 and 2 provide a sustained release relative to the reference solution as assessed by:
(1) a lower Cₘₐₓ for the formulations;
(2) a longer Tₘₐₓ for the formulations; and
(3) a longer time for which the blood morphine concentration was above 0.75 Cₘₐₓ for the formulations.

There was a significant decrease in Cₘₐₓ values for each formulation compared with the reference solution. The mean (±SD) Cₘₐₓ for the solution was 80.7 ± 26.4 ng/mL whereas the corresponding values for Formulation 1 and Formulation 2 formulations were 22.0 ± 8.1 ng/mL and 32.6 ± 18.1 ng/mL respectively. The variability in Cₘₐₓ for Molly 1 and 2 as demonstrated by the coefficient of variation was similar for all formulations. The Cₘₐₓ values for each formulation obtained under fed conditions were similar to the values obtained in the same patients under fasting conditions (Part A).

There was a significant increase in Tₘₐₓ values for the formulations relative to that obtained with the reference solution. The mean (±SD) Tₘₐₓ for solution was 1.32 ± 1.65 hours whereas the equivalent values were 5.83 ± 0.75 and 4.5 ± 0.84 hours for Formulation 1 and 2 respectively. The variability in Tₘₐₓ values for the formulations was less than that obtained for the solution. The Tₘₐₓ values were similar under fed and fasted conditions for each respective formulation.

The time the blood morphine concentration was greater than or equal to 0.75 Cₘₐₓ was significantly greater for the formulations compared to the reference solution dose. The mean time was 231.2 minutes for Formulation 1 and 168.5 minutes for Formulation 2 compared to only 52.2 minutes for the reference solution. Expressing these data as percentage of the time of the reference solution, Formulation 1 was 443% while Formulation 2 has 323% greater time that the blood morphine concentration was greater than 0.75 Cₘₐₓ compared to the solution. The data for the time greater than 0.75 Cₘₐₓ under fed and fasting conditions was similar for each respective formulation.

Under fed conditions, there was a significant difference between the AUC for the formulations and that obtained for the reference solution (Table 3.2) the reference solution having a greater AUC than either formulation. The mean areas were very similar for the formulations with mean values of 204.13 ± 106.11 ng.h/mL and 225.09 ± 138.52 ng.h/mL for Formulation 1 and Formulation 2 respectively. The mean AUC for the solution under fed conditions was 281.98 ± 112.58 ng.h/mL. The intersubject variability in AUC was similar for all formulations as shown by the coefficient of variation.

A comparison of AUC data obtained under fed and fasted conditions shows that the AUC for the reference solution expressed as a ratio of fed/fasted was 1.41 (range 0.94 to 1.9) with all but one patient having a ratio of greater than unity. There was a similar trend with the Formulations in that the mean AUC obtained when the formulations were administered immediately after food were larger than the equivalent value obtained in the fasted state.

The primary concern was to establish that "dose dumping" did not occur with either formulation. The data indicate that the bioavailability of morphine from formulations in the presence of food is at least equivalent to and possibly greater than the bioavailability from the same formulation in the fasted state and that the formulations behave in a similar manner to the solution with regard to the influence of food on the absorption of morphine.

The relative bioavailability for the formulations relative to that obtained for the reference solution was 79.4% for Formulation 1 and 78.2% for Formulation 2.

The AUC and relative bioavailability data suggest that the extent of absorption of morphine from the formulations is similar but slightly less than the solution in the fed state whereas the Cₘₐₓ, Tₘₐₓ and T_{≧0.75 Cmax} data indicate that the formulations exhibit the typical slower and prolonged absorption of a true sustained release preparation.

**TABLE 3.2**

| RESULT OF STUDY PART B | | | | | |
|---|---|---|---|---|---|
| PARAMETER | SOLUTION MEAN | MOLLY 1 | | MOLLY 2 | |
| | | MEAN | OBSERVED DIFF | MEAN | OBSERVED DIFF |
| AUC (ng.h/mL) | 281.98 | 204.13 | -77.85 | 225.09 | -56.89 |
| SD | ±112.58 | ±106.11 | | ±138.52 | |
| CV% | 40 | 52 | | 62 | |
| Cₘₐₓ (ng/mL) | 80.66 | 22.00 | -58.66 | 32.63 | -48.03 |
| SD | ±26.44 | ±8.05 | | ±18.07 | |
| CV% | 33 | 37 | | 55 | |
| Tₘₐₓ (hours) | 1.32 | 5.83 | 4.51 | 4.50 | 3.18 |
| SD | ±1.65 | ±0.75 | | ±0.84 | |
| CV% | 125 | 13 | | 19 | |
| Bioavailability (F%) | 100.0 | 79.4 | -20.6 | 78.2 | -21.8 |
| SD | ±0.00 | ±47.3 | | ±27.1 | |
| CV% | 0 | 60.0 | | 35.0 | |
| T_{≧0.75Cmax} (minutes) | 52.2 | 231.2 | 179.0 | 168.5 | 116.3 |
| SD | ±39.3 | ±73.9 | | ±55.5 | |
| CV% | 75 | 32 | | 33 | |

Finally, it is to be understood that various other modifications and/or alterations may be made without departing from the spirit of the present invention as outlined herein.

## Claims

1. A sustained release pharmaceutical pellet composition for administration to a patient at a predetermined dosage and interval which comprises a core element containing a therapeutically effective amount of at least one active ingredient having an aqueous solubility of at least 1 in 30 and a coating on said core element which comprises the following components:
(a) 1 to 85% by weight of a matrix polymer which is insoluble independent of pH.
(b) from 1 to 60% of an enteric polymer which is substantially insoluble at a pH of from 1 to 4, but which is soluble at a pH of from 6 to 7.5;
(c) from 1 to 60% of an acid-soluble compound soluble at a pH of from 1 to 4, sufficient to provide a slow rate of release of the active ingredient in the stomach;
said percentages being by weight based on the total weight of components (a), (b) and (c); the ratio of the components (a), (b) and (c) in said coating being effective such that a slow release of the active ingredient will occur in the stomach and a relatively constant faster release in the intestines such that blood levels of active ingredient are maintained within the therapeutic range over an extended period of time; the rate of release in the intestine being 1.2 to 3 times greater than in the stomach.

2. A sustained release pharmaceutical pellet composition according to claim 1 wherein the active ingredient is selected from antihistamines, antibiotics, antituberculosis agents, cholinergic agents, antimuscarinics, sympathomimetics, sympatholytic agents, autonomic drugs, iron preparations, haemostatics, cardiac drugs, antihypertensive agents, vasodilators, non-steroidal antiinflammatory agents, opiate agonists, anticonvulsants, tranquilisers, stimulants, barbiturates, sedatives, expectorants, antiemetics, gastrointestinal drugs, heavy metal antagonists, antithyroid agents, genitourinary smooth muscle relaxants and vitamins.

3. A sustained release pharmaceutical pellet composition according to claim 2 wherein the active ingredient is an opiate agonist selected from the group consisting of the salts of codeine, dextromoramide, hydrocodone, hydromorphine, pethidine, methadone, morphine and propoxyphene.

4. A sustained release pharmaceutical pellet composition according to claim 3 wherein the active ingredient is a morphine salt.

5. A sustained release pharmaceutical pellet composition according to claim 4 wherein the morphine salt is a morphine sulphate.

6. A sustained release pharmaceutical pellet composition according to claim 5 such that, in use, the time during which the morphine blood levels at steady state are greater than or equivalent to 75% of maximum blood levels (t≧0.75Cmax) is approximately 3 hours or greater.

7. A sustained release pharmaceutical pellet composition according to claim 1 such that, the active ingredient has a first dissolution profile measured at a pH of from 1 to 4, and a second dissolution profile measured at a pH of about 7.5 and such that said first and second dissolution profile are each at least equal to the minimum dissolution required to provide substantially the same bio-availability as with an immediate release oral dosage form.

8. A sustained release pharmaceutical pellet composition according to claim 1 wherein the coating contains:
as component (a), ethyl cellulose, acrylic ester polymers, methacrylic ester polymers or mixtures thereof, polymers or copolymers of acrylates or methacrylates having a low quaternary ammonium content including acrylic acid ethyl ester: methacrylic acid methyl ester (1:1) copolymer;
as component (b), cellulose acetate phthalate, hydroxypropyl methylcellulose phthalate, polyvinyl acetate phthalate, methacrylic acid:acrylic acid ethylester 1:1 copolymer, hydroxypropyl methylcellulose acetate succinate, shellac, cellulose acetate trimellitate and mixtures thereof; and
as component (c), polyvinyl pyrolidone, hydroxypropyl cellulose, hydroxypropyl methylcellulose, polyethylene glycol, polyvinyl alcohol and monomers therefor and mixtures thereof.

9. A sustained release pharmaceutical pellet composition according to claim 8, wherein the coating comprises:
35 to 75% by weight of component (a);
2 to 20% by weight of component (b); and
15 to 40% by weight of component (c).

10. A sustained release pharmaceutical pellet composition according to claim 8, wherein the coating also includes up to 50% of plasticizer selected from diethyl pthalate, triethyl citrate, triethyl acetyl citrate, triacetin, tributyl citrate, polyethylene glycol or glycerol and up to 75% of a filler selected from silicon dioxide, titanium dioxide, talc, alumina, starch, kaolin, polacrilin potassium, powdered cellulose, and microcrystalline cellulose and mixtures thereof, said percentages being based on the total weight of the coating.

11. A sustained release pharmaceutical pellet composition according to claim 10 wherein the coating contains:
| | |
|---|---|
| component (a) | 35 to 75% |
| component (b) | 2 to 20% |
| component (c) | 15 to 30% |
| plasticizer | 2.5 to 30% |

12. A sustained release pharmaceutical pellet composition according to any one of the preceding claims wherein the core element comprises an effective amount of at least one active ingredient; at least one core seed; and at least one binding agent.

13. A sustained release pharmaceutical pellet composition according to claim 12, wherein the core element comprises 0.1 to 94.9% by weight based on the total weight of the core element of an active ingredient; 5 to 99% by weight based on the total weight of the core element of core seeds; and 0.1 to 45% by weight based on the total weight of the core element of a binding agent selected from polyvinyl pyrrolidone, hydroxypropyl cellulose, hydroxypropyl methylcellulose, methylcellulose and hydroxyethyl cellulose, sugars and mixtures thereof.

14. A sustained release pharmaceutical pellet composition according to claim 13, wherein the core element has a formulation
| | |
|---|---|
| Morphine sulphate | 10 to 60% by weight |
| Core seeds | 30 to 89.9% by weight |
| Hydroxypropyl methylcellulose | 0.1 to 10% by weight |

15. A sustained release pharmaceutical pellet composition according to claim 13, wherein the core element has a formulation
| | |
|---|---|
| Morphine sulphate | 10 to 60% by weight |
| Core seeds | 30 to 87.5% by weight |
| Polyvinyl pyrrolidone | 2.5to 10% by weight |

16. A pharmaceutical sustained release product in a unit dosage form including a plurality of pellets, each pellet having the composition according to any one of the preceding claims.

17. A sustained release pharmaceutical pellet composition for administration to a patient at a predetermined dosage and interval which comprises a core element containing a therapeutically effective amount of an acid addition salt of morphine and a coating on said core element which comprises the following components:
(a) 1 to 85% by weight of a matrix polymer which is insoluble independent of pH.
(b) from 1 to 60% of an enteric polymer which is substantially insoluble at a pH of from 1 to 4, but which is soluble at a pH of from 6 to 7.5;
(c) from 1 to 60% of an acid-soluble compound soluble at a pH of from 1 to 4, sufficient to provide a slow rate of release of the active ingredient in the stomach;
said percentages being by weight based on the total weight of components (a), (b) and (c); the ratio of the components (a), (b) and (c) in said coating being effective such that a slow release of the active ingredient will occur in the stomach and a relatively constant faster release in the intestines such that blood levels of active ingredient are maintained within the therapeutic range over an extended period of time; the rate of release in the intestine being 1.2 to 3 times greater than in the stomach.

18. A sustained release pharmaceutical pellet composition according to claim 17 wherein said acid addition salt of morphine is morphine sulphate.

19. A sustained release pharmaceutical pellet composition according to claim 17 such that, in use, the time during which the plasma concentration is greater than or equivalent to 75% of maximum blood levels (t≧0.75Cmax) is at least 3 hours.

20. A sustained release pharmaceutical pellet composition according to claim 19 wherein the coating contains:
as component (a), ethyl cellulose, acrylic ester polymers, methacrylic ester polymers or mixtures thereof, polymers or copolymers of acrylates or methacrylates having a low quaternary ammonium content including acrylic acid ethyl ester: methacrylic acid methyl ester (1:1) copolymer;
as component (b), cellulose acetate phthalate hydroxypropyl methylcellulose phthalate, polyvinyl acetate phthalate, methacrylic acid: acrylic acid ethylester 1:1 copolymer, hydroxypropyl methylcellulose acetate succinate, shellac, cellulose acetate trimellitate and mixtures thereof; and
as component (c), polyvinylpyrolidone, hydroxypropyl cellulose, hydroxypropyl methylcellulose, polyethylene glycol, polyvinyl alcohol and monomers therefor and mixtures thereof.

21. A sustained release pharmaceutical pellet composition according to claim 20 wherein the coating comprises:
35 to 75% by weight of component (a);
2 to 20% by weight of component (b); and
15 to 40% by weight of component (c).

22. A method for preparing a sustained release pharmaceutical pellet composition, which method comprises providing
a core element including at least one active ingredient having an aqueous solubility of at least 1 in 30; and
a core coating composition comprising a solution , suspension or dispersion of
(a) 1 to 85% by weight of a matrix polymer which is insoluble independent of pH.
(b) from 1 to 60% of an enteric polymer which is substantially insoluble at a pH of from 1 to 4, but which is soluble at a pH of from 6 to 7.5;
(c) from 1 to 60% of an acid-soluble compound soluble at a pH of from 1 to 4, sufficient to provide a slow rate of release of the active ingredient in the stomach;
said percentages being by weight based on the total weight of components (a), (b) and (c);
introducing the core element into a fluidised bed reactor; and
spraying the core coating composition onto the core element;
the ratio of the components (a), (b) and (c) in said coating being effective such that a slow release of the active ingredient will occur in the stomach and a relatively constant faster release in the intestines such that blood levels of active ingredient are maintained within the therapeutic range over an extended period of time; the rate of release in the intestine being 1.2 to 3 times greater than in the stomach.

23. A method according to claim 22, which method further includes the preliminary step of providing
at least one active ingredient;
at least one binding agent;
at least one core seed; and
coating the core seed with the active ingredient and binding agent to form a core element.

24. A method according to claim 23, which method further includes the preliminary step of providing
at least one active ingredient;
at least one binding agent; and
an effective amount of a solvent,
mixing the ingredients; and
subjecting the ingredients to an extrusion followed by marumerisation to form a core element.

25. A method according to any one of claims 22 to 24 wherein the active ingredient is a morphine salt.

26. A sustained release pharmaceutical pellet composition according to claim 1 for use in treating pain- associated conditions.

27. A sustained release pharmaceutical pellet composition according to claim 26 wherein the pain associated conditions relate to the treatment of acute and chronic pain.

28. A sustained release pharmaceutical pellet composition according to claim 27 wherein the sustained release pharmaceutical pellet composition is provided in a unit dosage form which delivers an effective dose of active ingredient for a period of 8 to 24 hours.

## Patentansprüche

1. Arzneimittel mit Depotwirkung in Pillenform zur Verabreichung an einen Patienten bei vorgegebener Dosierung und zeitlicher Dauer, wobei die Pille ein Kernteil, das eine therapeutisch wirksame Menge eines aktiven Inhaltsstoffs enthält, das zumindest im Verhältnis 1 : 30 wasserlöslich ist, und eine Umhüllung des Kernelementes aufweist, die die folgenden Komponenten aufweist:
(a) 1 bis 85 Gew.-% eines Matrixpolymers, das unabhängig von dem pH-Wert unlöslich ist,
(b) 1 bis 60% enterales Polymer, das bei einem pH-Wert von 1 bis 4 im wesentlichen unlöslich, jedoch bei einem pH-Wert von 6 bis 7,5 löslich ist,
(c) zwischen 1 und 60% einer säurelöslichen Verbindung, die bei einem pH-Wert zwischen 1 und 4 löslich ist und ausreicht, um zu einer langsamen Freigabegeschwindigkeit des wirksamen Inhaltsstoffs in den Magen zu fuhren:
wobei die Prozentangaben gewichtsmäßig auf das Gesamtgewicht der Verbindung (a), (b) und (c) bezogen sind; das Verhältnis der Komponenten (a), (b) und (c) der Umhllung derart wirkt, daß eine langsame Freigabe des aktiven Inhaltsstoffs in dem Magen stattfindet und eine relativ konstante schnellere Freigabe in den Därmen, so daß die Blutwerte des aktiven Inhaltsstoffs über eine verlängerte Zeitspanne innerhalb des therapeutischen Bereiches gehalten sind; die Freigabegeschwindigkeit in den Darm 1,2 bis 3 mal größer ist als in den Magen.

2. Arzneimittel mit Depotwirkung in Pillenform, nach Anspruch 1, wobei der aktive Inhaltsstoff aus einer Gruppe ausgewählt ist, zu der Antihistamine, Antibiotica, antituberkulose Wirkstoffe, cholinergene (cholinergic) Wirkstoffe, Antimuscarine, Sympathomimetika, sympatholytische Wirkstoffe, autonomische Wirkstoffe, Eisenverbindungen, Haemostatika, Herzmedikamente, antihypertensive Wirkstoffe, Vasodilatoren, nicht-steroide entzündungshemmende Wirkstoffe, Opiatagonisten, Anticonvulsativa, Tranquilizer, Stimulanzien, Barbiturate, Sedativa, Expectoranzien, Antiemedica, gastrointestinale Wirkstoffe, Schwermetallantagonisten, Antithyroidwirkstoffe, Relaxativa für die glatte Urogenitalmuskulatur und Vitamine gehören.

3. Arzneimittel mit Depotwirkung in Pillenform, nach Anspruch 2, wobei der aktive Inhaltsstoff ein Opiatagonist ist, der aus einer Gruppe ausgewählt ist, zu der die Salze von Codein, Dextromoramid, Hydrocodon, Hydromorphin, Pethidin, Methadon, Morphin und Propoxyphen gehören.

4. Arzneimittel mit Depotwirkung in Pillenform, nach Anspruch 3, wobei der aktive Inhaltsstoff ein Morphinsalz ist.

5. Arzneimittel mit Depotwirkung in Pillenform, nach Anspruch 4, wobei das Morphinsalz ein Morphinsulfat ist.

6. Arzneimittel mit Depotwirkung in Pillenform, nach Anspruch 5, dadurch gekennzeichnet, daß bei der Verwendung die Zeitspanne, während der die Morphinblutwerte im stationären Zustand größer als oder gleich 75% des maximalen Blutwertes (t≧0,75 Cmax) sind, näherungsweise 3 Stunden oder mehr beträgt.

7. Arzneimittel mit Depotwirkung in Pillenform, nach Anspruch 1, dadurch gekenneichnet, daß der aktive Inhaltsstoff ein erstes Lösungsprofil, gemessen bei einem pH-Wert zwischen 1 und 4, und ein zweites Lösungsprofil, gemessen bei einem pH-Wert von etwa 7,5 aufweist, und daß das erste und das zweite Lösungsprofil jeweils wenigstens gleich der minimalen Lösung ist, die notwendig ist, um im wesentlichen dieselbe Bioverfügbarkeit zu erreichen, wie bei einer oralen Dosierungsform mit unmittelbarer Freisetzung.

8. Arzneimittel mit Depotwirkung in Pillenform, nach Anspruch 1, wobei die Umhüllung enthält:
als Komponente (a), Ethylzellulose, Acrylesterpolymere, Methacrylesterpolymere oder Mischungen davon, Polymere oder Copolymere von Acrylaten oder Methacrylaten mit einem niedrigen quaternären Ammoniumanteil einschließlich Acrylsäureethylester:Methacrylsäuremethylester-(1:1)-Copolymer;
als Komponente (b), Zelluloseacetatphthalat, Hydroxypropylmethylzellulosephthalat, Polyvinylacetatphthalat, Methacrylsäure:Acrylsäureethylester-(1:1)-Copolymer, Hydroxypropylmethylzelluloseacetatsuccinat, Schellack, Zelluloseacetattrimellitat und Mischungen davon; und
als Komponente (c), Polyvinylpyrrolidon, Hydroxyypropylzellulose, Hydroxypropylmethylzellulose, Polyethylenglykol, Polyvinylalkohol und Monomere hiervon sowie Mischungen daraus.

9. Arzneimittel mit Depotwirkung in Pillenform, nach Anspruch 8, wobei die Umhüllung enthält:
35 bis 75 Gewichtsprozent der Komponente (a);
2 bis 20 Gewichtsprozent der Komponente (b); und
15 bis 40 Gewichtsprozent der Komponente (c).

10. Arzneimittel mit Depotwirkung in Pillenform, nach Anspruch 8, wobei die Umhüllung außerdem bis zu 50% Plastifizierer, der aus der Gruppe ausgewählt ist, zu der Diethylpthalat, Triethylcitrat, Triethylacetylcitrat, Triacetin, Tributylcitrat, Polyethylenglykol oder Glyzerol, und bis zu 75% Füllstoff enthält,der aus einer Gruppe ausgewählt ist, die Siliziumdioxid, Titandioxid, Talkum, Aluminium, Stärke, Kaolin, Polyacrilinkalium, pulverförmige Zellulose und mikrokristalline Zellulose sowie Mischungen daraus enthält, wobei alle Gewichtsangaben auf dem Gesamtgewicht der Umhüllung basieren.

11. Arzneimittel mit Depotwirkung in Pillenform, nach Anspruch 10, wobei die Umhüllung enthält:
| | |
|---|---|
| Komponente (a) | 35 bis 75% |
| Komponente (b) | 2 bis 2O% |
| Komponente (c) | 15 bis 30% |
| Plastifizierer | 2,5 bis 30%. |

12. Arzneimittel mit Depotwirkung in Pillenform, nach einem der vorhergehenden Patentansprüche, wobei der Kernteil eine wirksame Menge wenigstens eines aktiven Inhaltsstoffs aufweist; wenigstens einen Keim für den Kern und wenigstens eine Bindemittel.

13. Arzneimittel in Pillenform mit andauernder Freigabe nach Anspruch 12, wobei das Kernteil 0,1 bis 94,9 Gewichtsprozente, bezogen auf das Gesamtgewicht des Kernelementes, an aktivem Inhaltsstoff, 5 bis 99 Gewichtsprozente Keimanteil für den Kern, bezogen auf das Gesamtgewicht des Kernelementes und 0,1 bis 45 Gewichtsprozente, bezogen auf das Gesamtgewicht des Kernelementes an Bindemittel enthält, das aus einer Gruppe ausgewählt ist, zu der Polyvinylpyrrolidon, Hydroxyoropylzellulose, Hydroypropylmethylzellulose, Methylzellulose und Hydroxyethylzellulose, Zucker und Mischungen hieraus gehören.

14. Arzneimittel mit Depotwirkung in Pillenform, nach Anspruch 13, wobei das Kernteil eine Zusammensetzung hat aus
| | |
|---|---|
| Morphinsulfat | 10 bis 60 Gew-% |
| Keimanteil für den Kern | 30 bis 89,9 Gew-% |
| Hydroxypropylmethylzellulose | 0,1 bis 10 Gew-%. |

15. Arzneimittel mit Depotwirkung in Pillenform, nach Anspruch 13, wobei das Kernteil eine Zusammensetzung hat aus
| | |
|---|---|
| Morphinsulfat | 10 bis 60 Gew-% |
| Keimanteil für den Kern | 30 bis 87,5 Gew-% |
| Polyvinylpyrrolidon | 2,5 bis 10 Gew-%. |

16. Arzneimittelprodukt mit Depotwirkung in Form einer Dosierungseinheit, zu der eine Vielzahl von Pillen gehören, wobei jede Pille eine Zusammensetzung nach einem oder mehreren der vorhergehenden Ansprüche aufweist.

17. Arzneimittel mit Depotwirkung in Pillenform zur Verabreichung an einen Patienten bei vorgegebener Dosierung und zeitlicher Dauer, wobei die Pille ein Kernteil, das eine therapeutisch wirksame Menge eines Säurezugabesalzes von Morphin und eine Umhüllung des Kernteils aufweist, die die folgenden Komponenten aufweist:
(a) 1 bis 85 Gew.-% eines Matrixpolymers, das unabhängig von dem pH-Wert unlöslich ist,
(b) 1 bis 60% enterales Polymer, das bei einem pH-Wert von 1 bis 4 im wesentlichen unlöslich, jedoch bei einem pH-Wert von 6 bis 7,5 löslich ist,
(c) zwischen 1 und 60% einer säurelöslichen Verbindung, die bei einem pH-Wert zwischen 1 und 4 löslich ist und ausreicht, um zu einer langsamen Freigabegeschwindigkeit des wirksamen Inhaltsstoffs in den Magen zu führen:
wobei die Prozentangaben gewichtsmäßig auf das Gesamtgewicht der Verbindung (a), (b) und (c) bezogen sind; das Verhältnis der Komponenten (a), (b) und (c) der Umhllung derart wirkt, daß eine langsame Freigabe des aktiven Inhaltsstoffs in dem Magen stattfindet und eine relativ konstante schnellere Freigabe in den Därmen, so daß die Blutwerte des aktiven Inhaltsstoffs über eine verlängerte Zeitspanne innerhalb des therapeutischen Bereiches gehalten sind; die Freigabegeschwindigkeit in den Darm 1,2 bis 3 mal größer ist als in den Magen.

18. Arzneimittel mit Depotwirkung in Pillenform, nach Anspruch 17, wobei das Säurezugabesalz von Morphin Morphinsulfat ist.

19. Arzneimittel mit Depotwirkung in Pillenform, nach Anspruch 17, dadurch gekennzeichnet, daß bei der Verwendung die Zeitspanne, während der die Plasmakonzentration größer als oder gleich 75% der maximalen Blutwerte (t≧0,75 Cmax) ist, näherungsweise 3 Stunden oder mehr beträgt.

20. Arzneimittel mit Depotwirkung in Pillenform, nach Anspruch 19, wobei die Umhüllung enthält:
als Komponente (a), Ethylzellulose, Acrylesterpolymere, Methacrylesterpolymere oder Mischungen davon, Polymere oder Copolymere von Acrylaten oder Methacrylaten mit einem niedrigen quaternären Ammoniumanteil einschließlich Acrylsäureethylester:Methacrylsäuremethylester-(1:1)-Copolymer;
als Komponente (b), Zelluloseacetatphthalat, Hydroxypropylmethylzellulosephthalat, Polyvinylacetatphthalat, Methacrylsäure:Acrylsäureethylester-(1:1)-Copolymer, Hydroxypropylmethylzelluloseacetatsuccinat, Schellack, Zelluloseacetattrimellitat und Mischungen davon; und
als Komponente (c), Polyvinylpyrrolidon, Hydroxyypropylzellulose, Hydroxypropylmethylzellulose, Polyethylenglykol, Polyvinylalkohol und Monomere hiervon sowie Mischungen daraus.

21. Arzneimittel mit Depotwirkung in Pillenform, nach Anspruch 20, wobei die Umhüllung enthält:
35 bis 75 Gewichtsprozent der Komponente (a);
2 bis 20 Gewichtsprozent der Komponente (b); und
15 bis 40 Gewichtsprozent der Komponente (c).

22. Verfahren zur Herstellung eines Arzneimittels mit mit Depotwirkung in Form von Pillen, wobei es zu dem Verfahren gehört, daß
ein Kernteil bereitgestellt wird, das wenigstens einen aktiven Inhaltsstoff aufweist, der eine Löslichkeit in Wasser von wenigstens 1 in 30 aufweist; und
daß eine Kernumhüllungszusammensetzung bereitgestellt wird, die eine Lösung, eine Suspension oder eine Dispersion von
(a) 1 bis 85 Gew-% eines Matrixpolymers enthält, das unabhängig vom pH-Wert unlöslich ist,
(b) 1 bis 60% eines enteralen Polymers enthält, das bei einem pH-Wert von 1 bis 4 im wesentlichen unlöslich, jedoch bei einem pH-Wert von 6 bis 7,5 löslich ist;
(c) 1 bis 60% einer säurelöslichen Verbindung enthält, die bei einem pH-Wert zwischen 1 und 4 löslich ist und ausreicht, um zu einer niedrigen Freigabegeschwindigkeit des aktiven Inhaltsstoffes in dem Magen zu sorgen;
wobei die Prozente Gewichtsprozente, basierend auf dem Gesamtgewicht aller Komponenten (a), (b) und (c) sind;
daß das Kernteil in einem Reaktor mit einem fluidisiertem Bett eingeführt wird und
daß die Kernumhüllungszusammensetzung auf das Kernelement aufgesprüht wird,
wobei das Verhältnis der Komponenten (a), (b) und (c) in der Umhüllung bewirkt, daß eine langsame Freigabe des wirksamen Inhaltsstoffes im Magen und eine verhältnismäßig konstante schnellere Freigabe in den Därmen stattfindet, so daß die Blutwerte des aktiven Inhaltsstoffes während einer verlängerten Zeitspanne innerhalb des therapeutischen Bereiches gehalten werden und die Freigabegeschwindigkeit in dem Darm 1,2 bis 3 mal größer ist als im Magen.

23. Verfahren nach Anspruch 22, zu dem ferner die vorbereitenden Schritte gehören, gemäß denen
wenigstens ein aktiver Inhaltsstoff;
wenigstens ein Bindemittel;
wenigstens ein Keim für den kern bereitgestellt
werden und
der Keim für den Kern mit dem aktiven Inhaltsstoff sowie dem Bindemittel beschichtet wird, um das Kernteil zu bilden.

24. Verfahren nach Anspruch 23, zu dem ferner die vorbereitenden Schritte gehören, gemäß denen
wenigstens ein aktiver Inhaltsstoff,
wenigstens ein Bindemittel und
eine wirksame Menge eines Lösungsmittels bereit
gestellt werden,
die Inhaltsstoffe gemischt werden, und
die Inhaltsstoffe einer Extrusion unterzogen werden, der eine Marumerisation folgt, um das Kernteil zu bilden.

25. Verfahren nach einem der Ansprüche 22 bis 24, wobei der aktive Inhaltsstoff ein Morphinsalz ist.

26. Arzneimittel mit Depotwirkung in Pillenform, nach Anspruch 1, zur Verwendung bei Schmerzzuständen.

27. Arzneimittel mit Depotwirkung in Pillenform, nach Anspruch 26, wobei die Schmerzzustände die Behandlung akuter und chronischer Schmerzen betreffen.

28. Arzneimittel mit Depotwirkung in Pillenform, nach Anspruch 27, wobei das Depotarzneimittel in Pillenform in einer Einheitsdosierform vorliegt, die eine wirksame Dosis des aktiven Inhaltsstoffes während einer Zeitspanne von 8 bis 24 Stunden abgibt.

## Revendications

1. Composition pharmaceutique sous forme de pastilles à libération prolongée, destinée à être administrée à un patient selon une posologie et un intervalle prédéterminés, comprenant une partie formant noyau contenant une quantité thérapeutiquement efficace d'au moins un ingrédient actif ayant une solubilité en milieu aqueux d'au moins 1 pour 30, et un enrobage de cette partie formant noyau, comprenant les composants suivants :
a) de 1 à 85 % en poids d'un polymère formant matrice, insoluble indépendamment du pH ;
b) de 1 à 60 % d'un polymère entérosoluble, à peu près insoluble à un pH de 1 à 4, mais soluble à un pH de 6 à 7,5 ;
c) de 1 à 60 % d'un composé soluble dans un acide à un pH de 1 à 4, suffisant pour obtenir une vitesse de libération lente de l'ingrédient actif dans l'estomac ;
ces pourcentages étant en poids par rapport au poids total des composants (a), (b) et (c) ; le rapport des composants (a), (b) et (c) dans l'enrobage étant efficace pour obtenir une libération lente de l'ingrédient actif dans l'estomac, et une libération plus rapide et relativement constante dans les intestins, de telle façon que les teneurs sanguines en ingrédient actif soient maintenues dans la plage thérapeutique pendant une période prolongée ; la vitesse de libération dans l'intestin étant de 1,2 à 3 fois supérieure à celle dans l'estomac.

2. Composition pharmaceutique sous forme de pastilles à libération prolongée, selon la revendication 1, dans laquelle l'ingrédient actif est choisi parmi les antihistaminiques, les antibiotiques, les agents antituberculeux, les agents cholinergiques, les antimuscariniques, les sympathomimétiques, les agents sympatholytiques, les médicaments pour le système neurovégétatif, les compositions à base de fer, les hémostatiques, les médicaments cardiaques, les agents antihypertensifs, les vasodilatateurs, les agents anti-inflammatoires non stéroïdiens, les agonistes opiacés, les anti-convulsivants, les tranquillisants, les stimulants, les barbituriques, les sédatifs, les expectorants, les anti-émétiques, les médicaments gastrointestinaux, les antagonistes de métal lourd, les agents anti-thyroïdiens, les relaxants de muscle lisse génito-urinaire et les vitamines.

3. Composition pharmaceutique sous forme de pastilles à libération prolongée selon la revendication 2, dans laquelle l'ingrédient actif est un agoniste opiacé choisi parmi les sels de codéine, le dextromoramide, l'hydrocodone, l'hydromorphine, la péthidine, la méthadone, la morphine et le propoxyphène.

4. Composition pharmaceutique sous forme de pastilles à libération prolongée selon la revendication 3, dans laquelle l'ingrédient actif est un sel de morphine.

5. Composition pharmaceutique sous forme de pastilles à libération prolongée selon la revendication 4, dans laquelle le sel de morphine est un sulfate de morphine.

6. Composition pharmaceutique sous forme de pastilles à libération prolongée selon la revendication 5, telle qu'en utilisation, la période pendant laquelle les teneurs sanguines en morphine à l'état stationnaire, sont supérieures ou équivalentes à 75 % des teneurs sanguines maximums (t ≧ 0,75 Cmax), est d'environ 3 heures ou plus.

7. Composition pharmaceutique sous forme de pastilles à libération prolongée selon la revendication 1, telle que l'ingrédient actif a un premier profil de dissolution déterminé à un pH de 1 à 4, et un deuxième profil de dissolution déterminé à un pH d'environ 7,5, et telle que ces premier et deuxième profils de dissolution, sont chacun au moins égal à la dissolution minimum nécessaire pour obtenir à peu près la même biodisponibilité qu'avec une forme pharmaceutique pour voie orale à libération immédiate.

8. Composition pharmaceutique sous forme de pastilles à libération prolongée selon la revendication 1, dans laquelle l'enrobage contient :
comme composant (a), de l'éthyl cellulose, des polymères d'ester acrylique, des polymères d'ester méthacrylique ou des mélanges de ceux-ci, des polymères ou des copolymères d'acrylates ou de méthacrylates ayant une faible teneur en groupes ammonium quaternaire, comprenant un copolymère (1:1) d'ester éthylique d'acide acrylique et d'ester méthylique d'acide méthacrylique ;
comme composant (b), de l'acétate-phtalate de cellulose, du phtalate d'hydroxypropyl méthylcellulose, de l'acétate-phtalate de polyvinyle, un copolymère 1:1 d'acide méthacrylique et d'ester éthylique d'acide acrylique, de l'acétate-succinate d'hydroxypropylméthylcellulose, de la gomme-laque, de l'acétate-trimellate de cellulose et des mélanges de ceux-ci ; et
comme composant (c), de la polyvinylpyrrolidone, de l'hydroxypropyl cellulose, de l'hydroxypropylméthyl cellulose, du polyéthylène glycol, du poly(alcool vinylique) et les monomères de ceux-ci ainsi que leurs mélanges.

9. Composition pharmaceutique sous forme de pastilles à libération prolongée selon la revendication 8, dans laquelle l'enrobage comprend :
de 35 à 75 % en poids du composant (a) ;
de 2 à 20 % en poids du composant (b) ; et
de 15 à 40 % en poids du composant (c).

10. Composition pharmaceutique sous forme de pastilles à libération prolongée selon la revendication 8, dans laquelle l'enrobage comprend également jusqu'à 50 % d'un plastifiant choisi parmi le phtalate de diéthyle, le citrate de triéthyle, l'acétyl citrate de triéthyle, la triacétine, le citrate de tributyle, le polyéthylène glycol ou le glycérol, et jusqu'à 75 % d'une charge choisie parmi le dioxyde de silicium, le dioxyde de titane, le talc, l'alumine, l'amidon, le kaolin, la polacriline potassique, la cellulose pulvérulente, et la cellulose microcristalline et leurs mélanges, ces pourcentages étant basés sur le poids total de l'enrobage.

11. Composition pharmaceutique sous forme de pastilles à libération prolongée selon la revendication 10, dans laquelle l'enrobage contient :
| | |
|---|---|
| composant (a) | 35 à 75 % |
| composant (b) | 2 à 20 % |
| composant (c) | 15 à 30 % |
| plastifiant | 2,5 à 30 % |

12. Composition pharmaceutique sous forme de pastilles à libération prolongée, selon l'une quelconque des revendications précédentes, dans laquelle la partie formant noyau comprend une quantité efficace d'au moins un ingrédient actif ; d'au moins un germe de noyau ; et d'au moins un agent de liaison.

13. Composition pharmaceutique sous forme de pastilles à libération prolongée selon la revendication 12, dans laquelle la partie formant noyau comprend de 0,1 à 94,9 % en poids par rapport au poids total de la partie formant noyau d'un ingrédient actif ; de 5 à 99 % en poids par rapport au poids total de la partie formant noyau, de germes de noyau ; et de 0,1 à 45 % en poids par rapport au poids total de la partie formant noyau, d'un agent de liaison choisi parmi la polyvinylpyrrolidone, l'hydroxypropyl cellulose, l'hydroxypropyl méthylcellulose, la méthylcellulose et l'hydroxyéthyl cellulose, les sucres et leurs mélanges.

14. Composition pharmaceutique sous forme de pastilles à libération prolongée selon la revendication 13, dans laquelle la partie formant noyau correspond à la composition :
| | |
|---|---|
| sulfate de morphine | 10 à 60 % en poids |
| germes de noyau | 30 à 89,9 % en poids |
| hydroxypropyl méthyl cellulose | 0,1 à 10 % en poids |

15. Composition pharmaceutique sous forme de pastilles à libération prolongée selon la revendication 13, dans laquelle la partie formant noyau correspond à la composition :
| | |
|---|---|
| sulfate de morphine | 10 à 60 % en poids |
| germes de noyau | 30 à 87,5 % en poids |
| polyvinyl pyrrolidone | 2,5 à 10 % en poids |

16. Produit pharmaceutique à libération prolongée sous forme pharmaceutique unitaire, comprenant plusieurs pastilles, chaque pastille ayant la composition de l'une quelconque des revendications précédentes.

17. Composition pharmaceutique sous forme de pastilles à libération prolongée, destinée à être administrée à un patient selon une posologie et un intervalle prédéterminés, comprenant une partie formant noyau contenant une quantité thérapeutiquement efficace d'un sel d'addition avec un acide dérivé de morphine, et un enrobage sur cette partie formant noyau, comprenant les composants suivants :
a) de 1 à 85 % en poids de polymère formant matrice qui est insoluble indépendamment du pH ;
b) de 1 à 60 % d'un polymère entérosoluble qui est à peu près insoluble à un pH de 1 à 4, mais qui est soluble à un pH de 6 à 7,5 ;
c) de 1 à 60 % d'un composé soluble dans un acide à un pH de 1 à 4, suffisant pour procurer une vitesse de libération lente de l'ingrédient actif dans l'estomac ;
ces pourcentages étant en poids par rapport au poids total des composants (a), (b) et (c) ; le rapport des composants (a), (b) et (c) dans l'enrobage étant efficace pour obtenir une libération lente de l'ingrédient actif dans l'estomac, et une libération plus rapide relativement constante dans les intestins, de telle façon que les teneurs sanguines en ingrédient actif, soient maintenues dans la plage thérapeutique pendant une période prolongée ; la vitesse de libération dans l'intestin étant de 1,2 à 3 fois supérieure à celle dans l'estomac.

18. Composition pharmaceutique sous forme de pastilles à libération prolongée selon la revendication 17, dans laquelle le sel d'addition avec un acide dérivé de morphine, est le sulfate de morphine.

19. Composition pharmaceutique sous forme de pastilles à libération prolongée selon la revendication 17, telle, qu'en utilisation, la période au cours de laquelle la concentration plasmatique est supérieure ou équivalente à 75 % des teneurs sanguines maximums (t ≧ 0,75 Cmax), est d'au moins 3 h.

20. Composition pharmaceutique sous forme de pastilles à libération prolongée selon la revendication 19, dans laquelle l'enrobage contient :
comme composant (a), de l'éthyl cellulose, des polymères d'ester acrylique, des polymères d'ester méthacrylique ou des mélanges de ceux-ci, des polymères ou des copolymères d'acrylates ou de méthacrylates ayant une teneur en groupes ammonium quaternaires faible, comprenant un copolymère (1:1) d'ester éthylique d'acide acrylique et d'ester méthylique d'acide méthacrylique ;
comme composant (b), de l'acétate-phtalate de cellulose, du phtalate d'hydroxypropyl méthylcellulose, de l'acétate-phtalate de polyvinyle, un copolymère (1:1) d'acide méthacrylique et d'ester éthylique d'acide acrylique, de l'acétate-succinate d'hydroxypropyl méthylcellulose, de la gomme-laque, de l'acétate-trimellate de cellulose et des mélanges de ceux-ci ; et
comme composant (c), de la polyvinylpyrrolidone, de l'hydroxypropyl cellulose, de l'hydroxypropyl méthylcellulose, du polyéthylène glycol, du poly(alcool vinylique) et leurs monomères ainsi que des mélanges de ceux-ci.

21. Composition pharmaceutique sous forme de pastilles à libération prolongée selon la revendication 20, dans laquelle l'enrobage comprend :
de 35 à 75 % en poids du composant (a) ;
de 2 à 20 % en poids du composant (b) ; et
de 15 à 40 % en poids du composant (c).

22. Procédé de préparation d'une composition pharmaceutique sous forme de pastilles à libération prolongée, selon lequel on prépare :
une partie formant noyau comprenant au moins un ingrédient actif ayant une solubilité en milieu aqueux d'au moins 1 pour 30 ; et
une composition d'enrobage de noyau comprenant une solution, une suspension ou une dispersion :
a) de 1 à 85 % en poids d'un polymère formant matrice insoluble indépendamment du pH ;
b) de 1 à 60 % d'un polymère entérosoluble à peu près insoluble à un pH de 1 à 4, mais soluble à un pH de 6 à 7,5 ;
c) de 1 à 60 % d'un composé soluble dans un acide à un pH de 1 à 4, suffisant pour obtenir une vitesse de libération lente de l'ingrédient actif dans l'estomac ;
ces pourcentages étant en poids par rapport au poids total des composants (a), (b) et (c) ;
on introduit la partie formant noyau dans un réacteur à lit fluidisé ; et
on pulvérise la composition d'enrobage de noyau sur la partie formant noyau ;
le rapport des composants (a), (b) et (c) dans l'enrobage étant efficace pour obtenir une vitesse lente de libération de l'ingrédient actif dans l'estomac, et une libération plus rapide et relativement constante dans les intestins, de telle façon que les teneurs sanguines en ingrédient actif soient maintenues dans la plage thérapeutique pendant une période prolongée ; la vitesse de libération dans l'intestin étant de 1,2 à 3 fois supérieure à celle dans l'estomac.

23. Procédé selon la revendication 22, comprenant en outre l'étape préliminaire de préparation :
d'au moins un ingrédient actif ;
d'au moins un agent de liaison ;
d'au moins un germe de noyau ; et
d'enrobage du germe de noyau avec l'ingrédient actif et l'agent de liaison pour former une partie formant noyau.

24. Procédé selon la revendication 23, comprenant en outre l'opération préliminaire de préparation :
d'au moins un ingrédient actif ;
d'au moins un agent de liaison ; et
d'une quantité efficace d'un solvant,
on mélange les ingrédients ; et
on soumet les ingrédients à une extrusion puis a leur façonnage en forme de sphères pour conduire à la partie formant noyau.

25. Procédé selon l'une quelconque des revendications 22 à 24, dans lequel l'ingrédient actif est un sel de morphine.

26. Composition pharmaceutique sous forme de pastilles à libération prolongée selon la revendication 1, destinée à être employée pour traiter des affections accompagnées de douleur .

27. Composition pharmaceutique sous forme de pastilles à libération prolongée selon la revendication 26, dans laquelle les affections accompagnées de douleur , relèvent du traitement de douleur aiguë et chronique.

28. Composition pharmaceutique sous forme de pastilles à libération prolongée selon la revendication 27, dans laquelle la composition pharmaceutique sous forme de pastilles à libération prolongée, est fournie sous forme pharmaceutique unitaire, procurant une dose efficace d'ingrédient actif pendant une période 8 à 24 h.
